(19) Europäisches Patentamt / European Patent Office / Office européen des brevets

(11) **EP 3 989 889 B1**

(12) **EUROPEAN PATENT SPECIFICATION**

(45) Date of publication and mention
of the grant of the patent:
**17.09.2025 Bulletin 2025/38**

(21) Application number: **20736571.9**

(22) Date of filing: **25.06.2020**

(51) International Patent Classification (IPC):
*A61F 5/44* (2006.01)    *A61F 5/443* (2006.01)
*A61F 5/445* (2006.01)    *A61F 5/448* (2006.01)

(52) Cooperative Patent Classification (CPC):
**A61F 5/4404; A61F 5/443; A61F 5/445; A61F 5/448**

(86) International application number:
**PCT/DK2020/050195**

(87) International publication number:
**WO 2020/259785 (30.12.2020 Gazette 2020/53)**

(54) **OSTOMY SYSTEM AND ELECTRODE ASSEMBLY WITH ANGULAR LEAKAGE DETECTION**

OSTOMIESYSTEM UND ELEKTRODENANORDNUNG MIT WINKELERKENNUNG VON LECKS

SYSTÈME DE STOMIE ET ASSEMBLAGE D'ÉLECTRODES AVEC DÉTECTION ANGULAIRES DES FUITES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2019 DK PA201970404**

(43) Date of publication of application:
**04.05.2022 Bulletin 2022/18**

(73) Proprietor: **Coloplast A/S
3050 Humlebaek (DK)**

(72) Inventors:
• **EMBORG, Jonas
3600 Frederikssund (DK)**
• **HANSEN, Jais Ask
3630 Jaegerspris (DK)**
• **LARSEN, Lars Erup
2760 Maaloev (DK)**
• **HVID, Niels
2950 Vedbaek (DK)**
• **SPEIERMANN, Finn
2830 Virum (DK)**
• **THOEGERSEN, Klaus
2920 Charlottenlund (DK)**

(56) References cited:
EP-A1- 1 991 187        EP-B1- 1 991 187
WO-A1-2015/014774    WO-A1-2019/120437
US-A1- 2019 142 623    US-B1- 6 171 289
US-B2- 7 737 321

EP 3 989 889 B1

## Description

The present disclosure relates to an electrode assembly for a base plate of an ostomy appliance and a base plate for an ostomy appliance. Thus, an electrode assembly/sensor assembly part for an ostomy appliance is disclosed. An ostomy system, and devices of the ostomy system are also disclosed. The ostomy system comprises one or more of an electrode assembly, a base plate for an ostomy appliance, an ostomy appliance and a monitor device. In particular, the present disclosure relates to electrode assembly/sensor assembly part, base plate, and ostomy appliance enabling or facilitating leakage classification and/or detection for an ostomy appliance and/or monitoring the operation of an ostomy appliance.

## Brief Description of the Drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.

Fig. 1 illustrates an exemplary ostomy system,
Fig. 2 illustrates an exemplary monitor device of the ostomy system,
Fig. 3 is an exploded view of a base plate of an ostomy appliance,
Fig. 4 is an exploded view of an exemplary electrode assembly,
Fig. 5 is a proximal view of parts of a base plate and/or sensor assembly part/electrode assembly,
Fig. 6 is a distal view of an exemplary electrode configuration,
Fig. 7 is a distal view of an exemplary masking element,
Fig. 8 is a distal view of an exemplary first adhesive layer,
Fig. 9 is a proximal view of the first adhesive layer of Fig. 8,
Fig. 10 is a distal view of a part of the base plate and/or sensor assembly part/electrode assembly including a monitor interface,
Fig. 11 is a distal view of the electrode configuration of Fig. 6,
Fig. 12 is a distal view of an exemplary electrode configuration,
Fig. 13 is a distal view of an exemplary masking element,
Fig. 14 is a distal view of an exemplary first adhesive layer,
Fig. 15 is a distal view of an exemplary electrode configuration,
Fig. 16 is a distal view of an exemplary electrode assembly,
Fig. 17 is a proximal view of an exemplary electrode assembly, and
Fig. 18 schematically illustrates an exemplary sensing zone configuration.

## Background

US 2019/142623 A1 relates to a thermoresponsive skin barrier assembly including a wound treatment assembly having a pump configured to expel a biosealant to a pump outlet port when subjected to a thermal stimulus, and an adhesive substrate layer for covering the wound. The adhesive substrate layer includes a conduit configured to transport the biosealant form the output port to the wound. The assembly further includes a control module in signal communication with a wound leakage sensor configured to activate a heating element disposed in proximity to the pump when wound leakage is detected.

## Detailed Description

The invention relates to an electrode assembly for a base plate of an ostomy appliance as defined in claim 1. Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated, or be implicit from the context that the "user" is not the "patient" him- or herself.

In the following, whenever referring to proximal side or surface of a layer, an element, a device or part of a device, the referral is to the skin-facing side or surface, when a user wears the ostomy appliance. Likewise, whenever referring to the distal side or surface of a layer, an element, a device or part of a device, the referral is to the side or surface facing away from the skin, when a user wears the ostomy appliance. In other words, the proximal side or surface is the side or surface closest to the user, when the appliance is fitted on a user and the distal side is the opposite side or surface - the side or surface furthest away from the user in use.

The axial direction is defined as the direction of the stoma, when a user wears the appliance. Thus, the axial direction is generally perpendicular to the skin or abdominal surface of the user.

A radial direction is defined as perpendicular to the axial direction. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The present disclosure relates to an electrode assembly for a base plate of an ostomy appliance, an ostomy system, and devices thereof, such as an ostomy appliance, a base plate for an ostomy appliance, a monitor device, and optionally one or more accessory devices. Further, methods related to the ostomy system and devices thereof are disclosed. An accessory device (also referred to as an external device) may be a mobile phone or other handheld device. An accessory device may be a personal electronic device, e.g. a wearable, such as a watch or other wrist-worn electronic device. An accessory device may be a docking station. The docking station may be configured to electrically and/or mechanically couple the monitor device to the docking station. The docking station may be configured for charging the monitor device and/or configured for transferring data between the monitor device and the docking station. The ostomy system may comprise a server device. The server device may be operated and/or controlled by the ostomy appliance manufacturer and/or a service centre.

The present disclosure provides an ostomy system and devices thereof, such as an ostomy appliance, a base plate for an ostomy appliance, a monitor device, and optionally one or more accessory devices which either alone or together facilitate reliable determination of the nature, severity, and rapidness of moisture propagation in or near the adhesive material provided for attaching the electrode assembly/base plate to the skin surface of a user. Depending on the nature of the pattern of moisture propagation in the adhesive, the ostomy system and devices thereof enable providing information to the user about the type of failure, and in turn enable providing an indication to the user of the severity and thus the remaining time frame for replacing the ostomy appliance without experiencing severe leakage and/or skin damage.

The ostomy appliance comprises a base plate and an ostomy pouch (also referred to as an ostomy bag). The ostomy appliance may be a colostomy appliance, an ileostomy appliance or a urostomy appliance. The ostomy appliance may be a two-part ostomy appliance, i.e. the base plate and the ostomy pouch may be releasably coupled e.g. with a mechanical and/or an adhesive coupling, e.g. to allow that a plurality of ostomy pouches can be utilized (exchanged) with one base plate. Further, a two-part ostomy appliance may facilitate correct application of the base plate to skin, e.g. to an improved user sight of the stomal region. The ostomy appliance may be a one-part ostomy appliance, i.e. the base plate and the ostomy pouch may be fixedly attached to each other. The base plate is configured for coupling to a user's stoma and/or skin surrounding the stoma, such as a peristomal skin area.

The ostomy appliance includes a base plate, such as a monolithic, one-piece base plate, e.g. integrated with a sensor assembly part/electrode assembly, or a base plate and a separate sensor assembly part/electrode assembly, such as a sensor assembly part to be subsequently applied to a base plate. For example, to allow an arbitrary base plate, such as a conventional base plate, to achieve the features as described herein. Features as described with respect to the base plate herein may be provided by a sensor assembly part/electrode assembly to be applied to a base plate, e.g. by the user. A sensor assembly part/electrode assembly may be adapted to adhere to an ostomy plate.

A disclosed method of attaching a base plate to a user's stoma and/or skin surrounding the stoma, such as the peristomal skin area, may comprise attaching a sensor assembly part/electrode assembly to a base plate and/or attaching the base plate, e.g. together with the attached sensor assembly part/electrode assembly, to the user's stoma and/or skin surrounding the stoma, such as the peristomal skin area. Alternatively, the method of attaching the base plate to the user's stoma and/or skin surrounding the stoma may comprise attaching the sensor assembly part/electrode assembly to the user's stoma and/or skin surrounding the stoma and attaching the base plate to the user's stoma and/or skin surrounding the stoma above the attached sensor assembly part. The method of attaching the base plate to the user's stoma and/or skin surrounding the stoma may comprise peeling of a base release liner of the base plate prior to attaching a sensor assembly part/electrode assembly to the base plate. The method of attaching the base plate to the user's stoma and/or skin surrounding the stoma may comprise peeling of an electrode assembly release liner, e.g. from the proximal adhesive layer, of the sensor assembly part/electrode assembly, e.g. prior to attaching the sensor assembly part/electrode assembly to the user's stoma and/or skin surrounding the stoma and attaching the base plate to the user's stoma and/or skin surrounding the stoma above the attached sensor assembly part.

An electrode assembly, e.g. for a base plate of an ostomy appliance or for a sensor patch, is disclosed, the electrode assembly comprising a support layer, the support layer having a stomal opening with a centre point. The electrode assembly comprises a plurality of electrodes including a first set of electrodes, the first set of electrodes comprising one or more first electrodes. The first set of electrodes comprises a first primary electrode, optionally a first secondary electrode, optionally a first tertiary electrode, and one or more first reference electrodes including a first primary reference electrode. The plurality of electrodes is optionally configured to detect presence of fluid in one or more of, such as all of, a first primary sensing zone, a first secondary sensing zone, and a first tertiary sensing zone. The first primary sensing zone is arranged in a first primary angle space, also denoted $V\_1\_1$, from the centre point of the support layer and/or at a first primary distance, also denoted $D\_1\_1$, from the centre point of the support layer, the first secondary sensing zone is optionally arranged in a first secondary angle space, also denoted $V\_1\_2$, from the centre point of the support layer and/or at a first secondary distance, also denoted $D\_1\_2$, from the centre point of the support layer, and the first tertiary sensing zone is optionally arranged in a first tertiary angle space, also denoted $V\_1\_3$, from the centre point of the support layer and/or at a first tertiary distance, also denoted $D\_1\_3$, from the centre point of the support layer.

Further, a base plate and/or a sensor assembly part for an ostomy appliance is disclosed, the base plate and/or sensor assembly part comprising a first adhesive layer with a proximal side configured for attachment of the base plate and/or the sensor assembly part to the skin surface of a user, the first adhesive layer having a stomal opening, such as a first adhesive stomal opening with a centre point; and an electrode assembly as disclosed herein, wherein the electrode assembly is arranged on the distal side of the first adhesive layer.

Also, an ostomy appliance comprising an electrode assembly as disclosed herein is provided. The ostomy appliance may comprise a base plate, wherein the electrode assembly is optionally embedded in or form an integrated part of the base plate. Further, an ostomy system comprising an ostomy appliance and an electrode assembly as disclosed herein is provided.

A base plate and/or a sensor assembly part for an ostomy appliance is disclosed, the base plate and/or the sensor assembly part comprising a first adhesive layer with a proximal side configured for attachment of the base plate and/or the sensor assembly part to the skin surface of a user, the first adhesive layer having a stomal opening, such as a first adhesive stomal opening, with a centre point; and a plurality of electrodes including a first leakage electrode, a second leakage electrode, and a third leakage electrode, wherein the plurality of electrodes is configured to detect presence of fluid on the proximal side of the first adhesive layer in a primary sensing zone and a secondary sensing zone. The primary sensing zone may be arranged in a primary angle space from the centre point of the first adhesive layer and/or the secondary sensing zone may be arranged in a secondary angle space from the centre point of the first adhesive layer. Alternatively or additionally, the primary sensing zone may be arranged in a primary radial space from the centre point of the first adhesive layer and the secondary sensing zone may be arranged in a secondary radial space from the centre point of the first adhesive layer.

Further, a monitor device for an ostomy system is disclosed. The ostomy system comprising an ostomy appliance with a base plate and/or a sensor assembly part/electrode assembly, such as the base plate and/or sensor assembly part as also described herein, such as a base plate and/or sensor assembly part having a first adhesive layer with a proximal side configured for attachment of the base plate and/or the sensor assembly part to the skin surface of a user, the first adhesive layer having a stomal opening with a centre point. The monitor device comprises a processor; memory; a first interface connected to the processor and the memory; and a second interface connected to the processor. The first interface is configured for obtaining ostomy data from the base plate and/or the sensor assembly part coupled to the first interface. The ostomy data comprises primary leakage ostomy data from a primary electrode set of the base plate and/or the sensor assembly part, and secondary leakage ostomy data from a secondary electrode set of the base plate and/or the sensor assembly part. The processor is configured to: obtain primary leakage parameter data based on the primary leakage ostomy data; obtain secondary leakage parameter data based on the secondary leakage ostomy data; detect presence of fluid on the proximal side of the first adhesive layer in a primary sensing zone based on the primary leakage parameter data, the primary sensing zone may be arranged in a primary angle space from the centre point of the first adhesive layer and/or arranged in a primary radial space from the centre point of the first adhesive layer; detect presence of fluid on the proximal side of the first adhesive layer in a secondary sensing zone based on the secondary leakage parameter data, the secondary sensing zone may be arranged in a secondary angle space from the centre point of the first adhesive layer and/or arranged in a secondary radial space from the centre point of the first adhesive layer; in accordance with a detection of presence of fluid in the primary sensing zone, transmit a primary leakage monitor signal comprising monitor data indicative of presence of fluid in the primary sensing zone via the second interface; and in accordance with a detection of presence of fluid in the secondary sensing zone, transmit a secondary leakage monitor signal comprising monitor data indicative of presence of fluid in the secondary sensing zone via the second interface.

In one or more exemplary electrode assemblies and/or base plates, the electrode assembly comprises a support member. The support member may comprise a base part and optionally a coupling part. The coupling part may be attached to the base part via a joint or hinge, e.g. formed by a weakened structure between the base part and the coupling part. In other words, the coupling part may be tiltably attached to the base part via a joint. The coupling part may be angled at least 15 degrees with respect to the base part.

In one or more exemplary electrode assemblies and/or base plates, the first set of electrodes is configured to detect presence of fluid in a first quaternary sensing zone, the first quaternary sensing zone arranged in a first quaternary angle space, also denoted $V\_1\_4$, from the centre point of the support layer and/or at a first quaternary distance ($D\_1\_4$) from the centre point of the support layer.

In one or more exemplary electrode assemblies and/or base plates, the first set of electrodes is configured to detect presence of fluid in a first quinary sensing zone, the first quinary sensing zone arranged in a first quinary angle space, also denoted $V\_1\_5$, from the centre point of the support layer and/or at a first quinary distance ($D\_1\_5$) from the centre point of the support layer.

In one or more exemplary electrode assemblies and/or base plates, the first set of electrodes is configured to detect presence of fluid in a first senary sensing zone, the first senary sensing zone arranged in a first senary angle space, also denoted $V\_1\_6$, from the centre point of the support layer and/or at a first senary distance ($D\_1\_6$) from the centre point of the support layer.

The electrode assembly and/or the base plate comprises a plurality of electrodes including a first set of electrodes, the first set of electrodes comprising one or more of a first primary electrode, a first secondary electrode, a first tertiary electrode, and one or more first reference electrodes including a first primary reference electrode.

The first primary reference electrode may comprise a first electrode branch with sensing part(s) arranged at a first distance from the centre point of the stomal opening. The first electrode branch may comprise sensing parts arranged for forming sensing points with sensing parts of electrodes of the first set of electrodes. The first primary reference electrode may comprise a second electrode branch with sensing part(s) arranged at a second distance from the centre point of the stomal opening. The second electrode branch may comprise sensing parts arranged for forming sensing points with sensing parts of electrodes of the second set of electrodes, such as primary sensing parts of the second primary electrode.

In one or more exemplary electrode assemblies and/or base plates, the first primary reference electrode comprises a primary sensing part or a plurality of primary sensing parts, the primary sensing part(s) arranged in the first primary angle space.

In one or more exemplary electrode assemblies and/or base plates, the first primary reference electrode comprises a secondary sensing part or a plurality of secondary sensing parts, the secondary sensing part(s) arranged in the first secondary angle space.

In one or more exemplary electrode assemblies and/or base plates, the first primary reference electrode comprises a tertiary sensing part or a plurality of tertiary sensing parts, the tertiary sensing part(s) arranged in the first tertiary angle space. The first primary reference electrode may comprise a quaternary sensing part or a plurality of quaternary sensing parts, the quaternary sensing part(s) arranged in the first quaternary angle space.

In other words, the first primary reference electrode (or sensing parts thereof) may span a plurality of angle spaces, such as the first secondary angle space and the first quaternary angle space.

In one or more exemplary electrode assemblies and/or base plates, the first primary electrode comprises a primary sensing part or a plurality of primary sensing parts, the primary sensing part(s) arranged in the first primary angle space. The first primary electrode may comprise a quaternary sensing part or a plurality of quaternary sensing parts, the quaternary sensing part(s) arranged in the first quaternary angle space.

In one or more exemplary electrode assemblies and/or base plates, the first secondary electrode comprises a secondary sensing part or a plurality of secondary sensing parts, the secondary sensing part(s) arranged in the first secondary angle space. The first secondary electrode may comprise a tertiary sensing part or a plurality of tertiary sensing parts, the tertiary sensing part(s) arranged in the first tertiary angle space. The first secondary electrode may comprise a quinary sensing part or a plurality of quinary sensing parts, the quinary sensing part(s) arranged in the first quinary angle space.

In one or more exemplary electrode assemblies and/or base plates, the first tertiary electrode comprises a tertiary sensing part or a plurality of tertiary sensing parts, the tertiary sensing part(s) arranged in the first tertiary angle space. The first tertiary electrode may comprise a senary sensing part or a plurality of senary sensing parts, the senary sensing part(s) arranged in the first senary angle space.

In one or more exemplary electrode assemblies and/or base plates, the one or more first reference electrodes may comprise a first secondary reference electrode and/or a first tertiary reference electrode. The first secondary reference electrode optionally comprises a secondary sensing part arranged in the first secondary angle space. The first secondary reference electrode may comprise a senary sensing part arranged in the first senary angle space. In other words, the first secondary reference electrode (or sensing parts thereof) may span a plurality of angle spaces, such as the first secondary angle space and the first senary angle space. The first tertiary reference electrode optionally comprises a tertiary sensing part arranged in the first tertiary angle space. The first tertiary reference electrode may comprise a quaternary sensing part arranged in the first quaternary angle space. In other words, the first tertiary reference electrode (or sensing parts thereof) may span a plurality of angle spaces, such as the first tertiary angle space and the first quaternary angle space.

In one or more exemplary electrode assemblies and/or base plates, the plurality of electrodes comprises a second set of electrodes, the second set of electrodes comprising a second primary electrode. The second primary electrode may be configured to detect presence of fluid in a second primary sensing zone, the second primary sensing zone arranged in a second primary angle space, also denoted V_2_1 from the centre point of the support layer and/or at a second primary distance, also denoted D_2_1) from the centre point of the support layer. In one or more exemplary electrode assemblies and/or base plates, the second primary distance is larger than the first primary distance. In one or more exemplary electrode assemblies and/or base plates, the second primary distance is larger than the first secondary distance. In one or more exemplary electrode assemblies and/or base plates, the second primary distance is larger than the first tertiary distance.

The second primary sensing zone of the electrode assembly and/or base plate is arranged in a second primary angle space from the centre point of the (stomal opening of) support layer. The second primary angle space may span a second primary angle in the range from 45° to 360°, such as in the range from 45° to 315°. In one or more exemplary electrode assemblies, the second primary angle space spans a second primary angle in the range from 45° to 315°, such as 60°±10°, 90°±10°, 120°±10°, 180°±10°, 270°±10°, or 300°±10°. The second primary angle may depend on the number of angular sensing zones on the electrode assembly/base plate. For example, the primary angle may be about 180° ± 15°, e.g. for an electrode assembly/base plate with two or more angular sensing zones. The second primary angle may be about 120° ± 15°, e.g. for an electrode assembly/base plate with two, three or more angular sensing zones. The second primary angle may be about 90° ± 15°, e.g. for an electrode assembly/base plate with two, three, four or more angular sensing zones.

The second primary sensing zone may be arranged in a second primary radial space, e.g. at a second primary distance, from the centre point of the (stomal opening of) support layer. The second primary radial space may span a second primary radius in the range from 25-60 mm, such as in the range from 30-55 mm. In other words, the second primary sensing zone may be arranged at a second primary distance, also denoted D_2_1, from the centre point of the support layer. The second primary distance may be in the range from 25-60 mm, such as in the range from 30-55 mm. For example, the primary sensing parts of the second primary electrode may be arranged, e.g. along a second primary arc, at a second primary distance, such as in the range from 25-60 mm, such as in the range from 30-55 mm, from the centre point of the support layer. The second primary distance may be larger than 30 mm.

In one or more exemplary electrode assemblies and/or base plates, the plurality of electrodes comprises a third set of electrodes, the third set of electrodes comprising a third primary electrode. The third primary electrode may be configured to detect presence of fluid in a third primary sensing zone, the third primary sensing zone arranged in a third primary angle space, also denoted V_3_1 from the centre point of the support layer and/or at a third primary distance, also denoted D_3_1 from the centre point of the support layer.

The third primary sensing zone of the electrode assembly and/or the base plate is arranged in a third primary angle space from the centre point of the (stomal opening of) support layer. The third primary angle space may span a third primary angle in the range from 45° to 360°, such as in the range from 45° to 315°. In one or more exemplary electrode assemblies, the third primary angle space spans a third primary angle in the range from 45° to 315°, such as 60°±10°, 90°±10°, 120°±10°, 180°±10°, 270°±10°, or 300°±10°. The third primary angle may depend on the number of angular sensing zones on the electrode assembly/base plate. For example, the third primary angle may be about 180° ± 15°, e.g. for an electrode assembly/base plate with two or more angular sensing zones. The third primary angle may be about 120° ± 15°, e.g. for an electrode assembly/base plate with two, three or more angular sensing zones. The third primary angle may be about 90° ± 15°, e.g. for an electrode assembly/base plate with two, three, four or more angular sensing zones.

The third primary sensing zone may be arranged in a third primary radial space, e.g. at a third primary distance, from the centre point of the (stomal opening of) support layer. The third primary radial space may span a third primary radius in the range from 15-55 mm, such as in the range from 20-50 mm, mm. In other words, the third primary sensing zone may be arranged at a third primary distance, also denoted $D\_3\_1$, from the centre point of the support layer. The third primary distance may be in the range from 15-55 mm, such as in the range from 20-50 mm. For example, the primary sensing parts of electrodes may be arranged, e.g. along a third primary arc, at a third primary distance, such as in the range from 15-55 mm, such as in the range from 20-50 mm, from the centre point of the support layer. The third primary distance may be larger than 30 mm.

In one or more exemplary electrode assemblies and/or base plates, the electrode assembly comprises a masking layer arranged on a proximal side of the support layer. The masking layer may cover at least parts of electrodes of the electrode assembly for forming conductor parts of electrodes.

The first primary sensing zone of the electrode assembly and/or the base plate is arranged in a first primary angle space from the centre point of the support layer. The first primary angle space may span a first primary angle in the range from 45° to 315°, such as in the range from 45° to 135°. In one or more exemplary electrode assemblies, the first primary angle space spans a first primary angle in the range from 45° to 315°, such as 60°±10°, 90°±10°, 120°±10°, or 180°±10°. The first primary angle may depend on the number of angular sensing zones on the electrode assembly/base plate. For example, the primary angle may be about 180° ± 15°, e.g. for an electrode assembly/base plate with two or more angular sensing zones. The first primary angle may be about 120° ± 15°, e.g. for an electrode assembly/base plate with two, three or more angular sensing zones. The first primary angle may be about 90° ± 15°, e.g. for an electrode assembly/base plate with two, three, four or more angular sensing zones.

The first primary sensing zone may be arranged in a first primary radial space, e.g. at a first primary distance, from the centre point of the (stomal opening of) support layer. The first primary radial space may span a first primary radius in the range from 10-50 mm, such as in the range from 20-45 mm. In other words, the first primary sensing zone may be arranged at a first primary distance, also denoted $D\_1\_1$, from the centre point of the support layer. The first primary distance may be in the range from 10-50 mm, such as in the range from 20-45 mm. For example, the primary sensing parts of electrodes may be arranged, e.g. along a first primary arc, at a first primary distance, such as in the range from 10-50 mm, such as in the range from 20-45 mm, from the centre point of the support layer.

The first secondary sensing zone may be arranged in a first secondary angle space from the centre point of the support layer. The first secondary angle space may span a first secondary angle in the range from 45° to 315°, such as in the range from 45° to 135°. In one or more exemplary electrode assemblies, the first secondary angle space spans a first secondary angle in the range from 45° to 315°, such as 60°±10°, 90°±10°, 120°±10°, or 180°±10°. The first secondary angle space may span a first secondary angle in the range from 45° to 315°, such as in the range from 45° to 135°. The first secondary angle may depend on the number of angular sensing zones on the electrode assembly/base plate. For example, the first secondary angle may be about 180° ± 15°, e.g. for an electrode assembly/base plate with two or more angular sensing zones. The first secondary angle may be about 120° ± 15°, e.g. for an electrode assembly/base plate with two, three or more angular sensing zones. The first secondary angle may be about 90° ± 15°, e.g. for an electrode assembly/base plate with two, three, four or more angular sensing zones.

The first secondary sensing zone may be arranged in a first secondary radial space, e.g. at a first secondary distance, from the centre point of the (stomal opening of) support layer. The first secondary radial space may span a first secondary radius in the range from 10-50 mm, such as in the range from 20-45 mm. In other words, the first secondary sensing zone may be arranged at a first secondary distance, also denoted $D\_1\_2$, from the centre point of the support layer. The first secondary distance may be in the range from 10-50 mm, such as in the range from 20-45 mm. For example, the secondary sensing parts of electrodes may be arranged, e.g. along a first secondary arc, at a first secondary distance, such as in the range from 10-50 mm, such as in the range from 20-45 mm, from the centre point of the support layer.

The plurality of electrodes may be configured to detect presence of fluid on the proximal side in a first tertiary sensing zone, the first tertiary sensing zone arranged in a first tertiary angle space from the centre point of the support layer. The first tertiary angle space may span a first tertiary angle in the range from 45° to 315°, such as in the range from 45° to 180°, for example in the range from 45° to 135°. In one or more exemplary electrode assemblies, the first tertiary angle space spans a first tertiary angle in the range from 45° to 180°, such as 60°±10°, 90°±10°, 120°±10°, or 180°±10°. The first tertiary angle may depend on the number of angular sensing zones on the electrode assembly/base plate. For example, the first tertiary angle may be about 180° ± 15°, e.g. for an electrode assembly/base plate with three or more angular sensing zones. The first tertiary angle may be about 120° ± 15°, e.g. for a base plate with three or more angular sensing zones. The first tertiary angle may be about 90° ± 15°, e.g. for a base plate with three, four or more angular sensing zones.

The first tertiary sensing zone may be arranged in a first tertiary radial space, e.g. at a first tertiary distance, from the centre point of the (stomal opening of) support layer. The first tertiary radial space may span a first tertiary radius in the range from 10-50 mm, such as in the range from 20-45 mm. In other words, the first tertiary sensing zone may be arranged at a first tertiary distance, also denoted $D\_1\_3$, from the centre point of the support layer. The first tertiary distance may be

EP 3 989 889 B1

in the range from 10-50 mm, such as in the range from 20-45 mm. For example, the tertiary sensing parts of electrodes may be arranged, e.g. along a first tertiary arc, at a first tertiary distance, such as in the range from 10-50 mm, such as in the range from 20-45 mm, from the centre point of the support layer.

In one or more exemplary electrode assemblies and/or base plates, the second distance(s), such as the second primary distance D_2_1 and/or a second secondary distance D_2_2, is/are larger than the first distance(s), such as the first primary distance D_1_1, the first secondary distance D_1_2 and the first tertiary distance D_1_3. In other words, the first primary electrode may be arranged on the inside of the second primary electrode. The first secondary electrode may be arranged on the inside of the second primary electrode. The first tertiary electrode may be arranged on the inside of the second primary electrode. The third primary electrode may be arranged on the inside of the second primary electrode and/or on the outside of the first primary electrode. The third primary electrode may be arranged on the outside of the first secondary electrode. The third primary electrode may be arranged on the outside of the first tertiary electrode.

In one or more exemplary electrode assemblies and/or base plates, the third distance(s), such as the third primary distance D_3_1 and/or a third secondary distance D_3_2, is/are larger than the first distance(s), such as the first primary distance D_1_1, the first secondary distance D_1_2 and the first tertiary distance D_1_3.

In one or more exemplary electrode assemblies, the first quaternary angle space spans a first quaternary angle in the range from 45° to 180°, such as 60°±10°, 90°±10°, 120°±10°, or 180°±10°. In one or more exemplary electrode assemblies, the first quinary angle space spans a first quinary angle in the range from 45° to 180°, such as 60°±10°, 90°±10°, 120°±10°, or 180°±10°. In one or more exemplary electrode assemblies, the first senary angle space spans a first senary angle in the range from 45° to 180°, such as 60°±10°, 90°±10°, 120°±10°, or 180°±10°.

The first quaternary sensing zone may be arranged in a first quaternary radial space, e.g. at a first quaternary distance, from the centre point of the (stomal opening of) support layer. The first quaternary radial space may span a first quaternary radius in the range from 10-50 mm, such as in the range from 20-45 mm. In other words, the first quaternary sensing zone may be arranged at a first quaternary distance, also denoted D_1_4, from the centre point of the support layer. The first quaternary distance may be in the range from 10-50 mm, such as in the range from 20-45 mm. For example, the quaternary sensing parts of electrodes may be arranged, e.g. along a first quaternary arc, at a first quaternary distance, such as in the range from 10-50 mm, such as in the range from 20-45 mm, from the centre point of the support layer.

The first quinary sensing zone may be arranged in a first quinary radial space, e.g. at a first quinary distance, from the centre point of the (stomal opening of) support layer. The first quinary radial space may span a first quinary radius in the range from 10-50 mm, such as in the range from 20-45 mm. In other words, the first quinary sensing zone may be arranged at a first quinary distance, also denoted D_1_5, from the centre point of the support layer. The first quinary distance may be in the range from 10-50 mm, such as in the range from 20-45 mm. For example, the quinary sensing parts of electrodes may be arranged, e.g. along a first quinary arc, at a first quinary distance, such as in the range from 10-50 mm, such as in the range from 20-45 mm, from the centre point of the support layer.

The first senary sensing zone may be arranged in a first senary radial space, e.g. at a first senary distance, from the centre point of the (stomal opening of) support layer. The first senary radial space may span a first senary radius in the range from 10-50 mm, such as in the range from 20-45 mm. In other words, the first senary sensing zone may be arranged at a first senary distance, also denoted D_1_6, from the centre point of the support layer. The first senary distance D_1_6 may be in the range from 10-50 mm, such as in the range from 20-45 mm. For example, the senary sensing parts of electrodes may be arranged, e.g. along a first senary arc, at a first senary distance, such as in the range from 10-50 mm, such as in the range from 20-45 mm, from the centre point of the support layer. In one or more exemplary electrode assemblies, the first distances are the same.

In one or more exemplary electrode assemblies and/or base plates, the first primary sensing zone and the first secondary sensing zone are separate, i.e. non-overlapping or substantially non-overlapping, sensing zones. The first primary sensing zone and the first tertiary sensing zone may be separate sensing zones, i.e. non-overlapping. The first secondary sensing zone and the first tertiary sensing zone may be separate sensing zones, i.e. non-overlapping. In one or more exemplary electrode assemblies, the first sensing zones are separate sensing zones.

The electrode assembly may comprise a proximal adhesive layer. The proximal adhesive layer may be arranged on the proximal side of the support layer and partly or fully covering the electrodes of the electrode assembly.

During use, the proximal adhesive layer or at least parts thereof adheres to the user's skin (peristomal area) and/or to additional seals, such as sealing paste, sealing tape and/or sealing ring. Thus, the proximal adhesive layer may be configured for attachment of the electrode assembly to the skin surface of a user. The proximal adhesive layer has a stomal opening with a centre point or is at least prepared for forming a stomal opening with a centre point. An electrode assembly according to the present disclosure enables detection of presence and angular position of fluid or output on the proximal side of the proximal adhesive layer (between a skin surface of the user and the proximal surface of the proximal adhesive layer). A proximal adhesive layer facilitates the use of the electrode assembly as an add-on to a base plate. The proximal adhesive layer will then after assembly with the base plate form a part of the adhesive securing attachment of the base plate to the skin of the user.

A sensing zone may be formed by or comprise electrodes or sensing parts thereof. The sensing parts of electrodes may be covered by the first adhesive layer/proximal adhesive layer and/or exposed to the surroundings.

The first primary sensing zone, the first secondary sensing zone, and/or the first tertiary sensing zone may cover sensing parts/electrodes embedded in the first adhesive layer as well as leakage electrodes/sensing parts being exposed to the surroundings. Thereby, the propagation or absorption of moisture in the first adhesive layer/proximal adhesive layer may be detected in one or more of the sensing zones, thereby providing for the determination of the direction of moisture propagation in the first adhesive layer/proximal adhesive layer and/or between the user's skin and the adhesive attaching the base plate to the user. Likewise, output propagating between the skin of the wearer and the first adhesive layer/proximal adhesive layer may be determined by the exposed electrodes. The electrodes/sensing parts may be exposed by means of sensor point openings as described below.

The proximal adhesive layer may be made of a skin composition. The skin composition may comprise one or more polyisobutenes and/or styrene-isoprene-styrene. The skin composition may comprise one or more hydrocolloids. The skin composition may comprise one or more water soluble or water swellable hydrocolloids.

The skin composition may be a pressure sensitive adhesive composition suitable for medical purposes comprising a rubbery elastomeric base and one or more water soluble or water swellable hydrocolloids. The skin composition may comprise one or more polybutenes, one or more styrene copolymers, one or more hydrocolloids, or any combination thereof. The combination of the adhesive properties of the polybutenes and the absorbing properties of the hydrocolloids renders the skin composition suitable for use in ostomy appliances/systems. The styrene copolymer may for example be a styrenebutadiene-styrene block copolymer or a styrene-isoprene-styrene block copolymer. Preferably, one or more styrene-isoprene-styrene (SIS) block type copolymers are employed. The amount of styrene block-copolymer may be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the first composition are selected from naturally occurring hydrocolloids, semisynthetic hydrocolloids, and synthetic hydrocolloids. The first composition may comprise 20-60% hydrocolloids. A preferred hydrocolloid is carboxymethyl cellulose (CMC). The skin composition may optionally contain other components, such as fillers, tackifiers, plasticizers, and other additives.

It is an advantage of the present disclosure that an optimum or improved use of an ostomy appliance is provided. In particular, the present disclosure facilitates that a base plate is not changed too late (leading to adhesive failure, leakage and/or skin damage), or at least that a user is informed that a leakage will happen, is happening, or has happened. Accordingly, the user or a health care professional is able to monitor and plan the use of the ostomy appliance.

Further, determination of moisture pattern types or angular leakage patterns is useful in helping to reduce the risk of a user experiencing leakage from an ostomy appliance. Further, determination of moisture pattern types and classification of operating states and/or leakage patterns of the ostomy appliance is further useful in helping reduce the risk of skin damage to a user.

The present disclosure provides an efficient, and easy-to-use ostomy appliance system with a high degree of comfort for a user.

The base plate and/or a sensor assembly part comprises a first adhesive layer. During use, the first adhesive layer or at least parts thereof adheres to the user's skin (peristomal area) and/or to additional seals, such as sealing paste, sealing tape and/or sealing ring. Thus, the first adhesive layer may be configured for attachment of the base plate and/or the sensor assembly part to the skin surface of a user. The first adhesive layer has a stomal opening, such as a first adhesive stomal opening with a centre point or is at least prepared for forming a stomal opening with a centre point. A base plate and/or a sensor assembly part according to the present disclosure enables detection of presence and angular position of fluid or output on the proximal side of the first adhesive layer (between a skin surface of the user and the proximal surface of the first adhesive layer).

The first adhesive layer may be made of a first composition. The first composition may comprise one or more polyisobutenes and/or styrene-isoprene-styrene. The first composition may comprise one or more hydrocolloids. The first composition may comprise one or more water soluble or water swellable hydrocolloids.

The first composition may be a pressure sensitive adhesive composition suitable for medical purposes comprising a rubbery elastomeric base and one or more water soluble or water swellable hydrocolloids. The first composition may comprise one or more polybutenes, one or more styrene copolymers, one or more hydrocolloids, or any combination thereof. The combination of the adhesive properties of the polybutenes and the absorbing properties of the hydrocolloids renders the first composition suitable for use in ostomy appliances. The styrene copolymer may for example be a styrene-butadienestyrene block copolymer or a styrene-isoprene-styrene block copolymer. Preferably, one or more styrene-isoprene-styrene (SIS) block type copolymers are employed. The amount of styrene block-copolymer may be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the first composition are selected from naturally occurring hydrocolloids, semisynthetic hydrocolloids, and synthetic hydrocolloids. The first composition may comprise 20-60% hydrocolloids. A preferred hydrocolloid is carboxymethyl cellulose (CMC). The first composition may optionally contain other components, such as fillers, tackifiers, plasticizers, and other additives.

The first primary electrode, such as a first electrode branch of the first primary electrode, may comprise one or more, such as a plurality of, primary sensing parts arranged in the first primary sensing zone. The number of primary sensing parts of the first primary electrode may be in the range from 3 to 20, such as in the range from 4 to 15 e.g. 4, 5, 6 or 7, or even larger than 20. The number of primary sensing parts may depend on the primary angle and/or the radial distance of primary sensing parts from the centre point. The first primary electrode may comprise one or more tertiary sensing parts arranged in the first tertiary sensing zone. The number of tertiary sensing parts of the first primary electrode may be in the range from 3 to 10, e.g. 4, 5, 6 or 7. The number of tertiary sensing parts of the first primary electrode may depend on the first tertiary angle and/or the radial distance of tertiary sensing parts from the centre point. The first primary electrode may comprise one or more quaternary sensing parts arranged in the first quaternary sensing zone. The number of quaternary sensing parts of the first primary electrode may be in the range from 3 to 10, e.g. 4, 5, 6 or 7. The number of quaternary sensing parts of the first primary electrode may depend on the first quaternary angle and/or the radial distance of quaternary sensing parts from the centre point.

The first secondary electrode comprises one or more secondary sensing parts arranged in the first secondary sensing zone. The number of secondary sensing parts of the first secondary electrode may be in the range from 3 to 10, e.g. 4, 5, 6 or 7. The number of secondary sensing parts of the first secondary electrode may depend on the first primary angle and/or the radial distance of secondary sensing parts from the centre point. The first secondary leakage electrode may comprise one or more tertiary sensing parts arranged in the first tertiary sensing zone. The number of tertiary sensing parts of the first secondary electrode may be in the range from 3 to 10, e.g. 4, 5, 6 or 7. The number of tertiary sensing parts of the first secondary electrode may depend on the first tertiary angle and/or the radial distance of tertiary sensing parts from the centre point. The first secondary leakage electrode may comprise one or more quinary sensing parts arranged in the first quinary sensing zone. The number of quinary sensing parts of the first secondary electrode may be in the range from 3 to 10, e.g. 4, 5, 6 or 7. The number of quinary sensing parts of the first secondary electrode may depend on the first quinary angle and/or the radial distance of quinary sensing parts from the centre point.

The first tertiary electrode may comprise one or more tertiary sensing parts arranged in the first tertiary sensing zone. The number of tertiary sensing parts of the first tertiary electrode may be in the range from 3 to 10, e.g. 4, 5, 6 or 7. The number of tertiary sensing parts of the first tertiary electrode may depend on the first tertiary angle and/or the radial distance of tertiary sensing parts from the centre point. The first tertiary leakage electrode may comprise one or more senary sensing parts arranged in the first senary sensing zone. The number of senary third sensing parts of the first tertiary electrode may be in the range from 3 to 10, e.g. 4, 5, 6 or 7. The number of senary sensing parts may depend on the tertiary angle and/or the radial distance of senary sensing parts from the centre point.

The proximal adhesive layer may have a plurality of sensor point openings. A sensor point opening of the proximal adhesive layer is configured to overlap a (sensing) part of an electrode, e.g. to form a sensor point. Sensing parts of the first primary electrode may be exposed to the proximal side of the proximal adhesive layer via sensor point openings in the proximal adhesive layer and/or the masking element. Sensing parts of the first secondary electrode may be exposed to the proximal side of the proximal adhesive layer via sensor point openings in the proximal adhesive layer and/or the masking element. Sensing parts of the first tertiary electrode may be exposed to the proximal side of the proximal adhesive layer via sensor point openings in the proximal adhesive layer and/or the masking element. Sensing parts of the first reference electrode(s) may be exposed to the proximal side of the proximal adhesive layer via sensor point openings in the proximal adhesive layer and/or the masking element.

A sensor point opening of the proximal adhesive layer may have a suitable shape and size facilitating access to a sensing part of an electrode from the proximal side of the proximal adhesive layer. A sensor point opening the proximal adhesive layer may have a circular or oval shape. A sensor point opening the proximal adhesive layer may have a shape of a rectangle or square optionally with rounded corners.

A minimum extension of a sensor point opening of the proximal adhesive layer may be at least 0.5 mm, such as at least 1 mm. A sufficiently large minimum extension reduces the risk of the proximal adhesive layer, due to the materials flow capabilities, closing the sensor point opening or at least partly or fully covering the sensing part of the corresponding electrode.

A maximum extension of a sensor point opening of the proximal adhesive layer may be less than 20 mm.

An exemplary sensor point opening of the proximal adhesive layer may have a minimum extension, e.g. measured radially from the centre point, in the range from 1 mm to 4 mm and/or a maximum extension, e.g. measured circumferentially around the centre point, in the range from 2 mm to 6 mm.

The sensor point openings of the proximal adhesive layer may comprise first primary sensor point openings arranged in the first primary angle space and/or at the first primary distance from the centre point. The number of first primary sensor point openings may depend on the first primary angle and/or the first primary distance (radial distance) of first primary sensor point openings from the centre point. In one or more exemplary electrode assemblies, the number of first primary sensor point openings is in the range from 5 to 20, such as in the range from 7 to 15. The first primary sensor point openings may comprise a first set of first primary sensor point openings and a reference set of first primary sensor point openings. The first primary sensor point openings of the proximal adhesive layer are configured to overlap respective primary sensing parts of electrodes, such as primary sensing parts of the first primary electrode and the first primary reference electrode. The first set of first primary sensor point openings may be configured to overlap respective primary sensing parts of the first primary electrode. The reference set of first primary sensor point openings may be configured to overlap respective primary sensing parts of the first primary reference electrode.

The sensor point openings of the proximal adhesive layer may comprise first secondary sensor point openings arranged in the first secondary angle space and/or at the first secondary distance from the centre point. The number of first secondary sensor point openings may depend on the first secondary angle and/or the radial distance of first secondary sensor point openings from the centre point. In one or more exemplary electrode assemblies, the number of first secondary sensor point openings is in the range from 5 to 20, such as in the range from 7 to 15. The first secondary sensor point openings may comprise a first set of first secondary sensor point openings and a reference set of first secondary sensor point openings. The first secondary sensor point openings of the proximal adhesive layer are configured to overlap respective secondary sensing parts of electrodes, such as secondary sensing parts of the first secondary electrode and the first primary reference electrode. The first secondary sensor point openings of the proximal adhesive layer may be configured to overlap respective secondary sensing parts of the first secondary electrode and the first secondary reference electrode.

The sensor point openings of the proximal adhesive layer may comprise first tertiary sensor point openings arranged in the first tertiary angle space and/or at the first tertiary distance from the centre point. The number of first tertiary sensor point openings may depend on the first tertiary angle and/or the radial distance of first tertiary sensor point openings from the centre point. In one or more exemplary electrode assemblies, the number of first tertiary sensor point openings is in the range from 5 to 20, such as in the range from 7 to 15. The first tertiary sensor point openings may comprise a first set of first tertiary sensor point openings and a reference set of first tertiary sensor point openings. The first tertiary sensor point openings of the proximal adhesive layer are configured to overlap respective tertiary sensing parts of electrodes, such as tertiary sensing parts of the first tertiary electrode and the first primary reference electrode. The first tertiary sensor point openings of the proximal adhesive layer may be configured to overlap respective tertiary sensing parts of the first tertiary electrode and a first reference electrode.

The sensor point openings of the proximal adhesive layer may comprise first quaternary sensor point openings arranged in the first quaternary angle space. The number of first quaternary sensor point openings may depend on the first quaternary angle and/or the radial distance of first quaternary sensor point openings from the centre point. In one or more exemplary electrode assemblies, the number of first quaternary sensor point openings is in the range from 5 to 20, such as in the range from 7 to 15. The first quaternary sensor point openings may comprise a first set of first quaternary sensor point openings and a reference set of first quaternary sensor point openings. The first quaternary sensor point openings of the proximal adhesive layer are configured to overlap respective quaternary sensing parts of electrodes.

The sensor point openings of the proximal adhesive layer may comprise first quinary sensor point openings arranged in the first quinary angle space. The number of first quinary sensor point openings may depend on the first quinary angle and/or the radial distance of first quinary sensor point openings from the centre point. In one or more exemplary electrode assemblies, the number of first quinary sensor point openings is in the range from 5 to 20, such as in the range from 7 to 15. The first quinary sensor point openings may comprise a first set of first quinary sensor point openings and a reference set of first quinary sensor point openings. The first quinary sensor point openings of the proximal adhesive layer are configured to overlap respective quinary sensing parts of electrodes.

The sensor point openings of the proximal adhesive layer may comprise first senary sensor point openings arranged in the first senary angle space. The number of first senary sensor point openings may depend on the first senary angle and/or the radial distance of first senary sensor point openings from the centre point. In one or more exemplary electrode assemblies, the number of first senary sensor point openings is in the range from 5 to 20, such as in the range from 7 to 15. The first senary sensor point openings may comprise a first set of first senary sensor point openings and a reference set of first senary sensor point openings. The first senary sensor point openings of the proximal adhesive layer are configured to overlap respective senary sensing parts of electrodes.

The sensor point openings of the proximal adhesive layer may comprise second primary sensor point openings arranged at a second primary distance from the centre point. The number of second primary sensor point openings may depend on the second primary distance of second primary sensor point openings from the centre point. In one or more exemplary electrode assemblies, the number of second primary sensor point openings is in the range from 5 to 100, such as in the range from 20 to 50. The second primary sensor point openings may comprise a second set of second primary sensor point openings and a reference set of second primary sensor point openings. The second primary sensor point openings of the proximal adhesive layer are configured to overlap respective primary sensing parts of electrodes, such as primary sensing parts of the second primary electrode and a reference electrode, such as the first primary reference electrode or a second primary reference electrode. The second set of second primary sensor point openings may be configured to overlap respective primary sensing parts of the second primary electrode. The reference set of second primary sensor point openings may be configured to overlap respective primary sensing parts of a reference electrode, such as the first primary reference electrode, the first secondary reference electrode or the second primary reference electrode.

The first adhesive layer may have a plurality of sensor point openings, e.g. for an ostomy appliance, where the electrode assembly is integrated or embedded in the base plate. A sensor point opening of the first adhesive layer is configured to overlap a (sensing) part of an electrode, e.g. to form a sensor point. Sensing parts of the first primary electrode may be exposed to the proximal side of the first adhesive layer via sensor point openings in the first adhesive layer and/or the masking element. Sensing parts of the first secondary electrode may be exposed to the proximal side of the first adhesive layer via sensor point openings in the first adhesive layer and/or masking element. Sensing parts of the first tertiary electrode may be exposed to the proximal side of the first adhesive layer via sensor point openings in the first adhesive layer and/or masking element. Sensing parts of the first reference electrode(s) may be exposed to the proximal side of the first adhesive layer via sensor point openings in the first adhesive layer and/or the masking layer.

A sensor point opening of the first adhesive layer may have a suitable shape and size facilitating access to an electrode/sensing part of an electrode from the proximal side of the first adhesive layer. A sensor point opening the first adhesive layer may have a circular or oval shape. A sensor point opening the first adhesive layer may have a shape of a rectangle or square optionally with rounded corners.

A distance between two neighbouring sensor point openings may be in the range from 1 mm to 20 mm, such as from 3 mm to 15 mm.

A minimum extension of a sensor point opening of the first adhesive layer may be at least 0.5 mm, such as at least 1 mm. A sufficiently large minimum extension reduces the risk of the first adhesive layer, due to the materials flow capabilities, closing the sensor point opening or at least partly or fully covering the sensing part of the corresponding leakage electrode.

A maximum extension of a sensor point opening of the first adhesive layer may be less than 20 mm.

An exemplary sensor point opening of the first adhesive layer may have a minimum extension, e.g. measured radially from the centre point, in the range from 1 mm to 4 mm and/or a maximum extension, e.g. measured circumferentially around the centre point, in the range from 2 mm to 6 mm.

The sensor point openings of the first adhesive layer may comprise first primary sensor point openings arranged in the first primary angle space and/or at the first primary distance from the centre point. The number of first primary sensor point openings may depend on the first primary angle and/or the first primary distance (radial distance) of first primary sensor point openings from the centre point. In one or more exemplary base plates, the number of first primary sensor point openings is in the range from 5 to 20, such as in the range from 7 to 15. The first primary sensor point openings may comprise a first set of first primary sensor point openings and a reference set of first primary sensor point openings. The first primary sensor point openings of the first adhesive layer are configured to overlap respective primary sensing parts of electrodes, such as primary sensing parts of the first primary electrode and the first primary reference electrode. The first set of first primary sensor point openings may be configured to overlap respective primary sensing parts of the first primary electrode. The reference set of first primary sensor point openings may be configured to overlap respective primary sensing parts of the first primary reference electrode.

The sensor point openings of the first adhesive layer may comprise first secondary sensor point openings arranged in the first secondary angle space and/or at the first secondary distance from the centre point. The number of first secondary sensor point openings may depend on the first secondary angle and/or the radial distance of first secondary sensor point openings from the centre point. In one or more exemplary base plates, the number of first secondary sensor point openings is in the range from 5 to 20, such as in the range from 7 to 15. The first secondary sensor point openings may comprise a first set of first secondary sensor point openings and a reference set of first secondary sensor point openings. The first secondary sensor point openings of the first adhesive layer are configured to overlap respective secondary sensing parts of electrodes, such as secondary sensing parts of the first secondary electrode and the first primary reference electrode. The first secondary sensor point openings of the proximal adhesive layer may be configured to overlap respective secondary sensing parts of the first secondary electrode and the first secondary reference electrode.

The sensor point openings of the first adhesive layer may comprise first tertiary sensor point openings arranged in the first tertiary angle space and/or at the first tertiary distance from the centre point. The number of first tertiary sensor point openings may depend on the first tertiary angle and/or the radial distance of first tertiary sensor point openings from the centre point. In one or more exemplary base plates, the number of first tertiary sensor point openings is in the range from 5 to 20, such as in the range from 7 to 15. The first tertiary sensor point openings may comprise a first set of first tertiary sensor point openings and a reference set of first tertiary sensor point openings. The first tertiary sensor point openings of the proximal adhesive layer are configured to overlap respective tertiary sensing parts of electrodes, such as tertiary sensing parts of the first tertiary electrode and the first primary reference electrode. The first tertiary sensor point openings of the proximal adhesive layer may be configured to overlap respective tertiary sensing parts of the first tertiary electrode and a first reference electrode.

The sensor point openings of the first adhesive layer may comprise first quaternary sensor point openings arranged in the first quaternary angle space and/or at the first quaternary distance from the centre point. The number of first quaternary sensor point openings may depend on the first quaternary angle and/or the radial distance of first quaternary sensor point openings from the centre point. In one or more exemplary base plates, the number of first quaternary sensor point openings is in the range from 5 to 20, such as in the range from 7 to 15. The first quaternary sensor point openings may comprise a first set of first quaternary sensor point openings and a reference set of first quaternary sensor point openings. The first quaternary sensor point openings of the proximal adhesive layer are configured to overlap respective quaternary sensing parts of electrodes.

The sensor point openings of the first adhesive layer may comprise first quinary sensor point openings arranged in the first quinary angle space and/or at the first quinary distance from the centre point. The number of first quinary sensor point openings may depend on the first quinary angle and/or the radial distance of first quinary sensor point openings from the centre point. In one or more exemplary base plates, the number of first quinary sensor point openings is in the range from 5 to 20, such as in the range from 7 to 15. The first quinary sensor point openings may comprise a first set of first quinary sensor point openings and a reference set of first quinary sensor point openings. The first quinary sensor point openings of the proximal adhesive layer are configured to overlap respective quinary sensing parts of electrodes.

The sensor point openings of the first adhesive layer may comprise first senary sensor point openings arranged in the first senary angle space and/or at the first senary distance from the centre point. The number of first senary sensor point openings may depend on the first senary angle and/or the radial distance of first senary sensor point openings from the centre point. In one or more exemplary base plates, the number of first senary sensor point openings is in the range from 5 to 20, such as in the range from 7 to 15. The first senary sensor point openings may comprise a first set of first senary sensor point openings and a reference set of first senary sensor point openings. The first senary sensor point openings of the proximal adhesive layer are configured to overlap respective senary sensing parts of electrodes.

The sensor point openings of the first adhesive layer may comprise second primary sensor point openings arranged at a second primary distance from the centre point. The number of second primary sensor point openings may depend on the second primary distance of second primary sensor point openings from the centre point. In one or more exemplary electrode assemblies, the number of second primary sensor point openings is in the range from 5 to 100, such as in the range from 20 to 50. The second primary sensor point openings may comprise a second set of second primary sensor point openings and a reference set of second primary sensor point openings. The second primary sensor point openings of the first adhesive layer are configured to overlap respective primary sensing parts of electrodes, such as primary sensing parts of the second primary electrode and a reference electrode, such as the first primary reference electrode or a second primary reference electrode. The second set of second primary sensor point openings may be configured to overlap respective primary sensing parts of the second primary electrode. The reference set of second primary sensor point openings may be configured to overlap respective primary sensing parts of a reference electrode, such as the first primary reference electrode (e.g. arranged in a second electrode branch of the first primary reference electrode, the first secondary reference electrode or the second primary reference electrode.

The first adhesive layer may have a substantially uniform thickness. The first adhesive layer may have a thickness in the range from 0.1 mm to 1.5 mm, e.g. in the range from 0.2 mm to 1.2 mm, such as 0.8 mm or 1.0 mm.

The first adhesive layer may have a primary thickness in a primary part of the first adhesive layer, e.g. in a primary region within a primary radial distance or in a primary radial distance range from the centre point of the stomal opening. The primary thickness may be in the range from 0.2 mm to 1.5 mm. such as about 1.0 mm. The primary radial distance may be in the range from 20 mm to 50 mm, such as in the range from 25 mm to 35 mm, e.g. 30 mm.

The first adhesive layer may have a secondary thickness in a secondary part of the first adhesive layer, e.g. in a secondary region outside a secondary radial distance or in a secondary radial distance range from the centre point of the stomal opening. The secondary thickness may be in the range from 0.2 mm to 1.0 mm, such as about 0.5 mm. The secondary radial distance may be in the range from 20 mm to 50 mm, such as in the range from 25 mm to 35 mm, e.g. 30 mm.

The base plate and/or the sensor assembly part may comprise a second layer. The second layer may be an adhesive layer. The second layer may have a second radial extension that is larger than a first radial extension of the first adhesive layer at least in a first angular range of the base plate and/or the sensor assembly part. Accordingly, a part of a proximal surface of the second layer may be configured for attachment to the skin surface of a user. The part of a proximal surface of the second layer configured for attachment to the skin surface of a user is also denoted the skin attachment surface of the second adhesive layer. The second layer may have a stomal opening, such as a second layer stomal opening and/or a second layer stomal opening and/or a second adhesive stomal opening, with a centre point.

The second adhesive layer may be made of a second composition. The second composition may comprise one or more polyisobutenes and/or styrene-isoprene-styrene. The second composition may comprise one or more hydrocolloids.

The second composition may be a pressure sensitive adhesive composition suitable for medical purposes comprising a rubbery elastomeric base and one or more water soluble or water swellable hydrocolloids. The second composition may comprise one or more polybutenes, one or more styrene copolymers, one or more hydrocolloids, or any combination thereof. The combination of the adhesive properties of the polybutenes and the absorbing properties of the hydrocolloids renders the second composition suitable for use in ostomy appliances. The styrene copolymer may for example be a styrenebutadiene-styrene block copolymer or a styrene-isoprene-styrene block copolymer. Preferably, one or more styrene-isoprene-styrene (SIS) block type copolymers are employed. The amount of styrene block-copolymer may be from 5% to 20% of the total adhesive composition. The butene component is suitably a conjugated butadiene polymer selected from polybutadiene, polyisoprene. The polybutenes are preferably present in an amount of from 35 - 50% of the total adhesive composition. Preferably, the polybutene is polyisobutylene (PIB). Suitable hydrocolloids for incorporation in the second composition are selected from naturally occurring hydrocolloids, semisynthetic hydrocolloids, and synthetic hydrocolloids. The second composition may comprise 20-60% hydrocolloids. A preferred hydrocolloid is carboxymethyl cellulose (CMC). The second composition may optionally contain other components, such as fillers, tackifiers, plasticizers, and other additives.

Different ratio of contents may change properties of the first and/or second adhesive layers. The second adhesive layer and the first adhesive layer may have different properties. The second adhesive layer (second composition) and the first adhesive layer (first composition) may have different ratios of polyisobutenes, styrene-isoprene-styrene, and/or hydrocolloids. For example, the second adhesive layer may provide a stronger attachment to the skin compared to attachment to the skin provided by the first adhesive layer. Alternatively, or additionally, the second adhesive layer may be thinner than the first adhesive layer. Alternatively, or additionally, the second adhesive layer may be less water and/or sweat absorbing than the first adhesive layer. Alternatively, or additionally, the second adhesive layer may be less moldable than the first adhesive layer. The second adhesive layer may provide a second barrier against leakage.

The second layer may have a substantially uniform thickness. The second layer may have a thickness in the range from 0.1 mm to 1.5 mm, e.g. in the range from 0.2 mm to 1.0 mm, such as 0.5 mm, 0.6 mm, or 0.7 mm.

In one or more exemplary base plates, the base plate comprises, e.g. as part of an electrode assembly, one or more electrodes, such as a plurality of electrodes, such as two, three, four, five, six, seven or more electrodes. The electrodes, e.g. some or all the electrodes, may be arranged on the distal side of the first adhesive layer and/or between the first adhesive layer and the second adhesive layer. The electrodes may be arranged in an electrode assembly, e.g. an electrode layer.

An electrode comprises a connection part for connecting the electrodes to other components and/or interface terminals/terminal elements. A connection part of an electrode may constitute an interface terminal/terminal element. An electrode may comprise one or more conductor parts and/or one or more sensing parts. The electrode assembly may be arranged between the first adhesive layer and the second adhesive layer or attached to the proximal surface of the first adhesive layer. The base plate, e.g. the electrode assembly, comprises a first set of electrodes, and one or more first reference electrodes including a first primary reference electrode. The first set of electrodes comprises one or more, such as all, of a first primary electrode, a first secondary electrode, and a first tertiary electrode.

The base plate, e.g. the electrode assembly, may comprise a second set of electrodes comprising one or more, such as all, of a second primary electrode, a second secondary electrode, and a second tertiary electrode.

A reference electrode, such as the first primary reference electrode, may be configured as or form a (common) reference electrode for some or all of the other electrodes of the electrode assembly.

The electrodes are electrically conductive and may comprise one or more of metallic (e.g. silver, copper, gold, titanium, aluminium, stainless steel), ceramic (e.g. ITO), polymeric (e.g. PEDOT, PANI, PPy), and carbonaceous (e.g. carbon black, carbon nanotube, carbon fibre, graphene, graphite) materials.

The first primary reference electrode may form a closed loop.

Two electrodes or sensing parts thereof may form a sensor. The first primary electrode and the first primary reference electrode may form a first primary sensor or first primary electrode pair for detecting presence of fluid in the first primary sensing zone. The first secondary electrode and the first primary reference electrode may form a first secondary sensor or first secondary leakage electrode pair for detecting presence of fluid in the first secondary sensing zone. The first tertiary electrode and the first primary reference electrode may form a first tertiary sensor or first tertiary electrode pair for detecting presence of fluid in the first tertiary sensing zone. The second primary electrode and the first primary reference electrode, e.g. sensing parts of second electrode branch of first primary reference electrode, may form a second primary sensor or second primary electrode pair for detecting presence of fluid in the second primary sensing zone. The second primary sensing zone may be arranged outside the first sensing zone(s).

An electrode may comprise a sensing part or a plurality of sensing parts, i.e. the part(s) of an electrode that are used for sensing. The first primary electrode may comprise a primary sensing part or a plurality of primary sensing parts. The primary sensing part(s) may contact the first adhesive layer and/or the proximal adhesive layer and may be arranged at least partly annularly around the stomal opening. The first primary electrode may comprise first primary conductor part(s), e.g. insulated from the proximal adhesive layer and/or the first adhesive layer, e.g. by a masking element/masking layer arranged between the first primary conductor part(s) and the first adhesive layer/the proximal adhesive layer. The primary sensing part(s) may extend or be distributed at least 270 degrees around the stomal opening, such as at least 300 degrees around the stomal opening. The first primary electrode may comprise a first primary connection part. A conductor part may be considered part of an electrode connecting two or more sensing parts, and/or connecting a sensing part with a connection part of the respective electrode. A sensing part may be considered a part of the electrode being suitable for sensing, e.g. liquid, such as liquid content, and/or output, such as output resulting from a leakage, or an imminent leakage. The sensing part may be suitable for sensing e.g. by its shape, said shape potentially being circular, oval, or rectangular. Thus, the conductor part may conduct a signal arising from the sensing part. An electrode may comprise alternating conductor parts and sensing parts.

The first secondary electrode may comprise a first secondary connection part. The first secondary electrode may comprise a secondary sensing part or a plurality of secondary sensing parts. The secondary sensing part(s) may contact the first adhesive layer and/or the proximal adhesive layer. The secondary sensing part(s) may be arranged at least partly annularly around the stomal opening. The first secondary electrode may comprise first secondary conductor part(s), e.g. insulated from the proximal adhesive layer and/or the first adhesive layer, e.g. by a masking element/masking layer arranged between the first secondary conductor part(s) and the first adhesive layer/the proximal adhesive layer. The secondary sensing part(s) may extend or be distributed at least 270 degrees around the stomal opening, such as at least 300 degrees around the stomal opening.

The base plate/electrode assembly may comprise a first tertiary electrode optionally comprising a third primary connection part. The first primary reference electrode may form a reference for the first tertiary electrode. The first tertiary electrode may comprise first tertiary conductor part(s), e.g. insulated from the proximal adhesive layer and/or the first adhesive layer, e.g. by a masking element/masking layer arranged between the first tertiary conductor part(s) and the first adhesive layer/proximal adhesive layer. The first tertiary electrode may comprise a tertiary sensing part or a plurality of tertiary sensing parts. The tertiary sensing part(s) may contact the first adhesive layer and/or the proximal adhesive layer. The tertiary sensing part(s) may be arranged at least partly annularly around the stomal opening. The tertiary sensing part(s) may extend or be distributed at least 270 degrees around the stomal opening, such as at least 300 degrees around the stomal opening.

The base plate may comprise a second adhesive layer, wherein the plurality of electrodes is arranged between the first adhesive layer and the second adhesive layer.

The electrode assembly may comprise a support layer, also denoted a support film. One or more electrodes may be formed, e.g. printed, on the proximal side of the support layer. One or more electrodes may be formed, e.g. printed, on the distal side of the support layer. Thus, one or more electrodes may be arranged between the support layer and the first adhesive layer or between the support layer and the proximal adhesive layer. The electrode assembly may have a stomal opening with a centre point. In the present context, it is to be understood that references to centre points of different layers, elements, assemblies, base plate etc. refers to a common centre point.

The support layer may comprise polymeric (e.g. polyurethane, PTFE, PVDF) and/or ceramic (e.g. alumina, silica) materials. In one or more exemplary base plates, the support layer is made of thermoplastic polyurethane (TPU). The support layer material may be made of or comprise one or more of polyester, a thermoplastic elastomer (TPE), polyamide, polyimide, Ethylene-vinyl acetate (EVA), polyurea, and silicones.

Exemplary thermoplastic elastomers of the support layer are styrenic block copolymers (TPS, TPE-s), thermoplastic polyolefin elastomers (TPO, TPE-o), thermoplastic Vulcanizates (TPV, TPE-v), thermoplastic polyurethanes (TPU), thermoplastic copolyester (TPC, TPE-E), and thermoplastic polyamides (TPA, TPE-A).

The electrode assembly/base plate may comprise a masking element or masking layer configured to insulate at least parts of the electrodes from the first adhesive layer of the base plate and/or from the proximal adhesive layer of the electrode assembly. The masking layer may comprise one or more, such as a plurality of, sensor point openings. The sensor point openings may comprise primary sensor point openings and/or secondary sensor point openings. The sensor point openings may comprise tertiary sensor point opening(s). The sensor point openings may comprise quaternary sensor point opening(s). The sensor point openings may comprise quinary sensor point opening(s). The sensor point openings may comprise senary sensor point opening(s).

The sensor point openings of the masking layer may comprise first primary sensor point openings arranged in the first primary angle space and/or at the first primary distance from the centre point. The number of first primary sensor point openings may depend on the first primary angle and/or the first primary distance (radial distance) of first primary sensor point openings from the centre point. In one or more exemplary masking layers, the number of first primary sensor point openings is in the range from 5 to 20, such as in the range from 7 to 15. The first primary sensor point openings may comprise a first set of first primary sensor point openings and a reference set of first primary sensor point openings. The first primary sensor point openings of the masking layer are configured to overlap respective primary sensing parts of electrodes, such as primary sensing parts of the first primary electrode and the first primary reference electrode. The first set of first primary sensor point openings may be configured to overlap respective primary sensing parts of the first primary electrode. The reference set of first primary sensor point openings may be configured to overlap respective primary sensing parts of the first primary reference electrode.

The sensor point openings of the masking layer may comprise first secondary sensor point openings arranged in the first secondary angle space and/or at the first secondary distance from the centre point. The number of first secondary sensor point openings may depend on the first secondary angle and/or the radial distance of first secondary sensor point openings from the centre point. In one or more exemplary masking layers, the number of first secondary sensor point openings is in the range from 5 to 20, such as in the range from 7 to 15. The first secondary sensor point openings may comprise a first set of first secondary sensor point openings and a reference set of first secondary sensor point openings. The first secondary sensor point openings of the masking layer are configured to overlap respective secondary sensing parts of electrodes, such as secondary sensing parts of the first secondary electrode and the first primary reference electrode. The first secondary sensor point openings of the masking layer may be configured to overlap respective secondary sensing parts of the first secondary electrode and the first secondary reference electrode.

The sensor point openings of the masking layer may comprise first tertiary sensor point openings arranged in the first tertiary angle space and/or at the first tertiary distance from the centre point. The number of first tertiary sensor point openings may depend on the first tertiary angle and/or the radial distance of first tertiary sensor point openings from the centre point. In one or more exemplary masking layers, the number of first tertiary sensor point openings is in the range from 5 to 20, such as in the range from 7 to 15. The first tertiary sensor point openings may comprise a first set of first tertiary sensor point openings and a reference set of first tertiary sensor point openings. The first tertiary sensor point openings of the masking layer are configured to overlap respective tertiary sensing parts of electrodes, such as tertiary sensing parts of the first tertiary electrode and the first primary reference electrode. The first tertiary sensor point openings of the proximal adhesive layer may be configured to overlap respective tertiary sensing parts of the first tertiary electrode and a first reference electrode.

The sensor point openings of the masking layer may comprise first quaternary sensor point openings arranged in the first quaternary angle space. The number of first quaternary sensor point openings may depend on the first quaternary angle and/or the radial distance of first quaternary sensor point openings from the centre point. In one or more exemplary masking layers, the number of first quaternary sensor point openings is in the range from 5 to 20, such as in the range from 7 to 15. The first quaternary sensor point openings may comprise a first set of first quaternary sensor point openings and a reference set of first quaternary sensor point openings. The first quaternary sensor point openings of the proximal adhesive layer are configured to overlap respective quaternary sensing parts of electrodes.

The sensor point openings of the masking layer may comprise first quinary sensor point openings arranged in the first quinary angle space. The number of first quinary sensor point openings may depend on the first quinary angle and/or the radial distance of first quinary sensor point openings from the centre point. In one or more exemplary masking layers, the number of first quinary sensor point openings is in the range from 5 to 20, such as in the range from 7 to 15. The first quinary sensor point openings may comprise a first set of first quinary sensor point openings and a reference set of first quinary sensor point openings. The first quinary sensor point openings of the proximal adhesive layer are configured to overlap respective quinary sensing parts of electrodes.

The sensor point openings of the masking layer may comprise first senary sensor point openings arranged in the first senary angle space. The number of first senary sensor point openings may depend on the first senary angle and/or the radial distance of first senary sensor point openings from the centre point. In one or more exemplary masking layers, the number of first senary sensor point openings is in the range from 5 to 20, such as in the range from 7 to 15. The first senary sensor point openings may comprise a first set of first senary sensor point openings and a reference set of first senary sensor point openings. The first senary sensor point openings of the proximal adhesive layer are configured to overlap respective senary sensing parts of electrodes.

The sensor point openings of the masking layer may comprise second primary sensor point openings arranged at a second primary distance from the centre point. The number of second primary sensor point openings may depend on the second primary distance of second primary sensor point openings from the centre point. In one or more exemplary masking layers, the number of second primary sensor point openings is in the range from 5 to 100, such as in the range from 20 to 50. The second primary sensor point openings may comprise a second set of second primary sensor point openings and a reference set of second primary sensor point openings. The second primary sensor point openings of the proximal adhesive layer are configured to overlap respective primary sensing parts of electrodes, such as primary sensing parts of the second primary electrode and a reference electrode, such as the first primary reference electrode or a second primary reference electrode. The second set of second primary sensor point openings may be configured to overlap respective primary sensing parts of the second primary electrode. The reference set of second primary sensor point openings may be configured to overlap respective primary sensing parts of a reference electrode, such as the first primary reference electrode, the first secondary reference electrode or the second primary reference electrode.

The masking layer may comprise one or more, such as a plurality of, terminal openings. A terminal opening may overlap with one or more connection parts of electrodes. In one or more exemplary base plates/electrode assemblies, each terminal opening overlaps with a single connection part of an electrode.

The masking layer may comprise polymeric (e.g. polyurethane, PTFE, PVDF) and/or ceramic (e.g. alumina, silica) materials. In one or more exemplary base plates and/or electrode assemblies, the masking layer is made of or comprises thermoplastic polyurethane (TPU). In one or more exemplary base plates and/or electrode assemblies, the masking layer is made of or comprises polyester. The masking layer material may be made of or comprise one or more of polyester, a thermoplastic elastomer (TPE), polyamide, polyimide, Ethylene-vinyl acetate (EVA), polyurea, and silicones.

Exemplary thermoplastic elastomers of the masking layer are styrenic block copolymers (TPS, TPE-s), thermoplastic polyolefin elastomers (TPO, TPE-o), thermoplastic Vulcanizates (TPV, TPE-v), thermoplastic polyurethanes (TPU), thermoplastic copolyester (TPC, TPE-E), and thermoplastic polyamides (TPA, TPE-A).

The base plate may comprise a first intermediate element. The first intermediate element may be arranged between the electrodes/electrode layer and the first adhesive layer and/or between the second layer and the first adhesive layer. The first intermediate element may be made of an insulating material.

The base plate may comprise a base release liner covering the first adhesive layer. The base release liner is a protective layer that protects adhesive layer(s) during transport and storage and is peeled off by the user prior to applying the base plate on the skin. The base release liner may have a stomal opening with a centre point.

The electrode assembly may comprise an electrode assembly release liner, e.g. covering proximal adhesive layer of the electrode assembly if present. The electrode assembly release liner is a protective layer that protects adhesive layer(s) during transport and storage and is peeled off by the user prior to applying the electrode assembly on the skin. The electrode assembly release liner may have a stomal opening with a centre point.

The base plate may comprise a top layer. The top layer is a protective layer protecting the adhesive layer(s) from external strains and stress when the user wears the ostomy appliance. The electrodes, e.g. some or all the electrodes, may be arranged between the first adhesive layer and the top layer. The top layer may have a stomal opening with a centre point. The top layer may have a thickness in the range from 0.01 mm to 1.0 mm, e.g. in the range from 0.02 mm to 0.2 mm, such as 0.04 mm. The top layer may have a stomal opening with a centre point.

The base plate and/or the electrode assembly may comprise a support member and/or a monitor interface. The support member may form at least part of the monitor interface. The monitor interface may be configured for electrically and/or mechanically connecting the electrode assembly and/or ostomy appliance to the monitor device. The monitor interface may be configured for wirelessly connecting the electrode assembly and/or ostomy appliance to the monitor device. Thus, the monitor interface of the base plate and/or electrode assembly is configured to electrically and/or mechanically couple the ostomy appliance and the monitor device.

The support member of the electrode assembly may comprise, e.g. as part of a first connector of the monitor interface, a coupling part for forming a mechanical connection, such as a releasable coupling between the monitor device and the electrode assembly and/or base plate. The coupling part may be configured to engage with a coupling part of the monitor device for releasably coupling the monitor device to the electrode assembly and/or base plate.

The monitor interface of the base plate may comprise, e.g. as part of a first connector of the monitor interface, a plurality of terminals, such as two, three, four, five, six, seven or more terminals, for forming electrical connections with respective terminals of the monitor device. The monitor interface may comprise one or more reference terminals including a first primary reference terminal and/or a first secondary reference terminal. The monitor interface may comprise a first primary terminal and/or a second primary terminal. The monitor interface optionally comprises a first secondary terminal and/or a first tertiary terminal. The monitor interface may comprise a third primary terminal. In one or more exemplary electrode assemblies/base plates, the monitor interface has M terminals, wherein M is an integer in the range from 4 to 8.

Connection parts of electrodes of the electrode assembly or terminal elements connected thereto may form respective terminals of the monitor interface. The first primary connection part of first primary electrode may form the first primary terminal of the monitor interface. The first secondary connection part of the first secondary electrode may form the first secondary terminal of the monitor interface. The first tertiary connection part of the first tertiary electrode may form the first tertiary terminal of the monitor interface. The first primary reference connection part of the first primary reference electrode may form the first primary reference terminal. A first secondary reference connection part of the first primary reference electrode may form the first secondary reference terminal. The second primary connection part of second primary electrode may form the second primary terminal of the monitor interface. The third primary connection part of third primary electrode may form the third primary terminal of the monitor interface.

The base plate may comprise a coupling ring or other coupling member for coupling an ostomy pouch to the base plate (two-part ostomy appliance). The centre point may be defined as a centre of the coupling ring.

The base plate and/or the sensor assembly part has a stomal opening with a centre point. The stomal opening of the base plate and/or the electrode assembly may be formed collectively of stomal opening(s) of the layers of the base plate and/or the electrode assembly, such as of the top layer, the first adhesive layer, the second layer and/or the electrode assembly (support layer and optionally proximal adhesive layer). The stomal opening(s) of the layers of the base plate and/or the electrode assembly part, such as of the top layer, the first adhesive layer, the second layer and/or the electrode assembly may be aligned to form the stomal opening of the base plate and/or the electrode assembly. The stomal opening may be a through-going passage of the base plate and/or the electrode assembly. The stomal opening may be arranged substantially in the centre of the base plate and/or the electrode assembly. The stomal opening(s) of the layers of the base plate and/or the electrode assembly may be arranged substantially in the centre of the respective layer. The stomal opening may be configured to receive a stoma of the user and/or the stomal opening may be configured to allow output from the stoma to pass through the stomal opening an into an ostomy pouch attached to the base plate. For example, the stomal opening may be configured to allow passage of output from a proximal side of the base plate and/or electrode assembly to a distal side of the base plate and/or electrode assembly. The size and/or shape of the stomal opening may typically be adjusted by the user or nurse before application of the ostomy appliance to accommodate the user's stoma. In one or more exemplary base plates, the user forms the stomal opening during preparation of the base plate for application.

The plurality of electrodes of the electrode assembly is optionally configured to detect presence of fluid in one or more of, such as all of, a second primary sensing zone, a second secondary sensing zone, and a second tertiary sensing zone. The second primary sensing zone is arranged in a second primary angle space, also denoted $V\_2\_1$, from the centre point of the support layer and/or at a second primary distance, also denoted $D\_2\_1$, from the centre point of the support layer. The second secondary sensing zone is optionally arranged in a second secondary angle space, also denoted $V\_2\_2$, from the centre point of the support layer and/or at a second secondary distance, also denoted $D\_2\_2$, from the centre point of the support layer, and the second tertiary sensing zone is optionally arranged in a second tertiary angle space, also denoted $V\_2\_3$, from the centre point of the support layer and/or at a second tertiary distance, also denoted $D\_2\_3$, from the centre point of the support layer.

In one or more exemplary electrode assemblies, the second primary angle space partly overlaps, e.g. with at least 30° or at least 45°, the first primary angle space. In other words, the first primary angle space and the second primary angle space may be offset e.g. by a primary offset angle. For example, where the first primary angle space and the second primary angle space each span about 120°, the first primary angle space and the second primary angle space may be offset by a primary offset angle of 60°. In one or more exemplary electrode assemblies, the second primary angle space partly overlaps the first tertiary angle space and/or the first secondary angle space.

In one or more exemplary electrode assemblies, the second secondary angle space partly overlaps, e.g. with at least 30° or at least 45°, the first secondary angle space. In one or more exemplary electrode assemblies, the second secondary angle space partly overlaps, e.g. with at least 30° or at least 45°, the first primary angle space and/or the first tertiary angle space.

In one or more exemplary electrode assemblies, the second tertiary angle space partly overlaps, e.g. with at least 30° or at least 45°, the first tertiary angle space. In one or more exemplary electrode assemblies, the second tertiary angle space partly overlaps, e.g. with at least 30° or at least 45°, the first primary angle space and/or the first secondary angle space.

The monitor device comprises a processor and one or more interfaces, such as a first interface and/or a second interface. The monitor device may comprise a memory for storing ostomy data.

In one or more exemplary monitor devices, the processor is configured to apply a processing scheme, the first interface is connected to the processor and the memory, and the first interface is configured for collecting ostomy data from the base plate and/or electrode assembly coupled to the first interface.

The ostomy data may comprise one or more, such as all, of first primary ostomy data from a first primary electrode pair of the electrode assembly/base plate, first secondary ostomy data from a first secondary electrode pair of the electrode assembly/base plate, and first tertiary ostomy data from a first tertiary electrode pair of the electrode assembly/base plate. A second interface is connected to the processor. To apply a processing scheme may comprise one or more of obtain first primary parameter data based on the first primary ostomy data; obtain first secondary parameter data based on the first secondary ostomy data; and obtain first tertiary parameter data based on the first tertiary ostomy data. To apply a processing scheme may comprise to determine an operating state of the electrode assembly/base plate of the ostomy appliance based on one or more, such as all, of the first primary parameter data, the first secondary parameter data and the first tertiary parameter data. The operating state may be indicative of the presence of output in a sensing zone and/or a degree of radial erosion of the base plate, such as of the first adhesive layer, and/or an acute leakage risk for the ostomy appliance. The monitor device is optionally configured to, in accordance with a determination that the operating state is a first primary operating state, transmit a first primary monitor signal comprising monitor data indicative of the first primary operating state of the base plate via the second interface; and/or in accordance with a determination that the operating state is a primary secondary operating state, transmit a first secondary monitor signal comprising monitor data indicative of the first secondary operating state of the base plate via the second interface.

In one or more exemplary monitor devices, the ostomy data comprises first primary ostomy data from a first primary electrode pair of electrodes, e.g. the first primary electrode and the first primary reference electrode.

In one or more exemplary monitor devices, the ostomy data comprises first secondary ostomy data from a first secondary electrode pair of electrodes, e.g. the first secondary electrode and the first primary reference electrode. The first secondary electrode and a first secondary reference electrode may constitute the first secondary electrode pair.

In one or more exemplary monitor devices, the ostomy data comprises first tertiary ostomy data from a first tertiary electrode pair of electrodes, e.g. the first tertiary electrode and the first primary reference electrode. The first secondary electrode and the first primary reference electrode may constitute the first tertiary electrode pair. The first tertiary electrode and a first tertiary reference electrode may constitute the first tertiary electrode pair.

In one or more exemplary monitor devices, the ostomy data comprises first quaternary ostomy data from a first quaternary electrode pair of electrodes, e.g. the first quaternary electrode and the first primary reference electrode. The first primary electrode and the first secondary reference electrode may constitute the first quaternary electrode pair.

In one or more exemplary monitor devices, the ostomy data comprises first quaternary ostomy data from a first quaternary electrode pair of electrodes, e.g. the first quaternary electrode and the first primary reference electrode. The first primary electrode and the first secondary reference electrode may constitute the first quaternary electrode pair. The first tertiary reference electrode and the first primary reference electrode may constitute the first quaternary electrode pair.

In one or more exemplary monitor devices, the ostomy data comprises first quinary ostomy data from a first quinary electrode pair of electrodes, e.g. the first secondary electrode and the first primary reference electrode.

In one or more exemplary monitor devices, the ostomy data comprises first senary ostomy data from a first senary electrode pair of electrodes, e.g. the first tertiary electrode and the first secondary reference electrode.

Thus, the present disclosure allows angular detection of leakage/moisture in up to six angular spaces around the centre point.

The first primary parameter data, the first secondary parameter data, and the first tertiary parameter data may be indicative of resistance between the first primary electrode pair, the first secondary electrode pair, and the first tertiary electrode pair, respectively.

The first primary parameter data, the first secondary parameter data, and the first tertiary parameter data may be indicative of a rate of change in resistance between the first electrode pair, the second electrode pair, and the third electrode pair, respectively.

In one or more exemplary monitor devices, to determine an operating state of the base plate is based on a first primary criteria set based on the first primary parameter data and/or the first secondary parameter data, wherein the operating state is determined to be the first primary operating state if the first primary criteria set is satisfied. The first primary criteria set may comprise one or more first primary criteria based on one or more of first primary parameter data, first secondary parameter data and first tertiary parameter data. The first primary criteria set may comprise a first primary criterion based on the first primary parameter data. The first primary criteria set may comprise a further first primary criterion based on the first secondary parameter data. The first primary criteria set may comprise an even further first primary criterion based on the first tertiary parameter data.

In one or more exemplary monitor devices, to determine an operating state of the base plate may be based on one or more first primary thresholds.

The first primary criteria set may be given by or comprise

$$(P\_1\_1 < TH\_1\_1),$$

$$(P\_1\_2 > TH\_1\_1),$$

and

$$(P\_1\_3 > TH\_1\_1),$$

wherein P_1_1 is a first primary parameter based on the first parameter data, TH_1_1 is a first primary threshold value, P_1_2 is a first secondary parameter based on the first secondary parameter data, and P_1_3 is a first tertiary parameter based on the first tertiary parameter data, and wherein the first primary operating state is indicative of output on the proximal side of the base plate/electrode assembly in the first primary sensing zone.

In one or more exemplary monitor devices, to determine an operating state of the base plate is based on a first secondary criteria set based on the first primary parameter data and/or the first secondary parameter data, wherein the operating state is determined to be the first secondary operating state if the first secondary criteria set is satisfied. The first secondary criteria set may comprise one or more first secondary criteria based on one or more of first primary parameter data, first secondary parameter data and first tertiary parameter data. The first secondary criteria set may comprise a first secondary criterion based on the first secondary parameter data. The first secondary criteria set may comprise a further first secondary criterion based on the first primary parameter data. The first secondary criteria set may comprise an even further first secondary criterion based on the first tertiary parameter data.

In one or more exemplary monitor devices, to determine an operating state of the base plate may be based on one or more first secondary thresholds.

The first secondary criteria set may be given by or comprise

$$(P\_1\_1 > TH\_1\_2),$$

$$(P\_1\_2 < TH\_1\_2),$$

and

$$(P\_1\_3 > TH\_1\_2),$$

wherein P_1_1 is a first primary parameter based on the first parameter data, TH_1_2 is a first secondary threshold value, P_1_2 is a first secondary parameter based on the first secondary parameter data, and P_1_3 is a first tertiary parameter based on the first tertiary parameter data, and wherein the first secondary operating state is indicative of output on the proximal side of the base plate/electrode assembly in the first secondary sensing zone.

In one or more exemplary monitor devices, to determine an operating state of the base plate is based on a first tertiary criteria set based on the first tertiary parameter data and/or the first secondary parameter data, wherein the operating state is determined to be the first tertiary operating state if the first tertiary criteria set is satisfied. The first tertiary criteria set may comprise one or more first tertiary criteria based on one or more of first primary parameter data, first secondary parameter data and first tertiary parameter data. The first tertiary criteria set may comprise a first tertiary criterion based on the first tertiary parameter data. The first tertiary criteria set may comprise a further first tertiary criterion based on the first primary parameter data. The first tertiary criteria set may comprise an even further first tertiary criterion based on the first tertiary parameter data.

In one or more exemplary monitor devices, to determine an operating state of the base plate may be based on one or more first tertiary thresholds.

The first tertiary criteria set may be given by or comprise

$$(P\_1\_1 > TH\_1\_3),$$

$$(P\_1\_2 > TH\_1\_3),$$

and

$$(P\_1\_3 < TH\_1\_2),$$

wherein P_1_1 is a first primary parameter based on the first parameter data, TH_1_3 is a first tertiary threshold value, P_1_2 is a first secondary parameter based on the first secondary parameter data, and P_1_3 is a first tertiary parameter based on the first tertiary parameter data, and wherein the first tertiary operating state is indicative of output on the proximal side of the base plate/electrode assembly in the first tertiary sensing zone.

In one or more exemplary monitor devices, to determine an operating state of the base plate is based on a default criteria set based on the first primary parameter data, the first secondary parameter data, and the first tertiary parameter data, wherein the operating state is determined to be the default operating state if the default criteria set is satisfied, and in accordance with a determination that the operating state is the default operating state, transmit a default monitor signal comprising monitor data indicative of the default operating state of the ostomy appliance.

The default criteria set may be given by

$$(P\_1\_1 > TH\_D),$$

$$(P\_1\_2 > TH\_D),$$

and

$$(P\_1\_3 > TH\_D)$$

wherein P_1_1 is a first primary parameter based on the first primary parameter data and indicative of the resistance between the first primary electrode pair, TH_D is a default threshold value, P_1_2 is a first secondary parameter based on the first secondary parameter data and indicative of the resistance between the first secondary electrode pair, P_1_3 is a first tertiary parameter based on the first tertiary parameter data and indicative of the resistance between the first tertiary electrode pair, and wherein the default operating state is indicative of very low or no leakage of output in the first primary sensing zone, the first secondary sensing zone, and the first tertiary sensing zone.

To apply a processing scheme may comprise to obtain first primary parameter data based on first primary ostomy data of the ostomy data and determine an operating state of the base plate of the ostomy appliance based on the first primary parameter data. The monitor device may be configured to, in accordance with a determination that the operating state is a first primary operating state, transmit a first primary monitor signal comprising monitor data indicative of the first primary operating state of the ostomy appliance.

In one or more exemplary monitor devices, the first primary operating state of the base plate corresponds to a situation, wherein the first primary electrode pair detects fluid, such as output, between the electrode assembly and the skin of the user in the first primary sensing zone. The first primary monitor signal may be indicative of a high risk of leakage or presence of output in the first primary sensing zone.

The first primary criteria set may comprise or be given by

$$(P\_1\_1 < TH\_1\_1)$$

wherein P_1_1 is a first primary parameter based on the first primary parameter data and indicative of the resistance between electrodes of the first primary electrode pair, TH_1_1 is a first primary threshold value, and wherein the first primary operating state is indicative of high risk of leakage or presence of output in the first primary sensing zone of the electrode assembly/base plate/ostomy appliance.

To apply a processing scheme may comprise to obtain first secondary parameter data based on first secondary ostomy data of the ostomy data and determine an operating state of the base plate of the ostomy appliance based on the first secondary parameter data. The monitor device may be configured to, in accordance with a determination that the operating state is a first secondary operating state, transmit a first secondary monitor signal comprising monitor data indicative of the first secondary operating state of the ostomy appliance.

In one or more exemplary monitor devices, the first secondary operating state of the base plate corresponds to a situation, wherein the first secondary electrode pair detects fluid, such as output, between the electrode assembly and the skin of the user in the first secondary sensing zone. The first secondary monitor signal may be indicative of a high risk of leakage or presence of output in the first secondary sensing zone.

The first secondary criteria set may comprise or be given by

$$(P\_1\_2 < TH\_1\_2)$$

wherein P_1_2 is a first secondary parameter based on the first secondary parameter data. The first secondary parameter may be indicative of the resistance between electrodes of the first secondary electrode pair, TH_1_2 is a first secondary threshold value, and the first secondary operating state is indicative of high risk of leakage or presence of output in the first secondary sensing zone of the electrode assembly/base plate/ostomy appliance.

To apply a processing scheme may comprise to obtain first tertiary parameter data based on first tertiary ostomy data of the ostomy data and determine an operating state of the base plate of the ostomy appliance based on the first tertiary parameter data. The monitor device may be configured to, in accordance with a determination that the operating state is a first tertiary operating state, transmit a first tertiary monitor signal comprising monitor data indicative of the first tertiary operating state of the ostomy appliance.

In one or more exemplary monitor devices, the first tertiary operating state of the base plate corresponds to a situation, wherein the first tertiary electrode pair detects fluid, such as output, between the electrode assembly and the skin of the user in the first tertiary sensing zone. The first tertiary monitor signal may be indicative of a high risk of leakage or presence of output in the first tertiary sensing zone.

The first tertiary criteria set may comprise or be given by

$$(P\_1\_3 < TH\_1\_3)$$

wherein P_1_3 is a first tertiary parameter based on the first tertiary parameter data and indicative of the resistance between electrodes of the first tertiary electrode pair, TH_1_3 is a first tertiary threshold value, and the first tertiary operating state is indicative of high risk of leakage or presence of output in the first tertiary sensing zone of the electrode assembly/base plate/ostomy appliance.

To apply a processing scheme may comprise to obtain first quaternary parameter data based on first quaternary ostomy data (from first quaternary electrode pair) of the ostomy data, and determine an operating state of the base plate of the ostomy appliance based on the first quaternary parameter data. The monitor device may be configured to, in accordance with a determination that the operating state is a first quaternary operating state, transmit a first quaternary monitor signal comprising monitor data indicative of the first quaternary operating state of the ostomy appliance.

In one or more exemplary monitor devices, the first quaternary operating state of the base plate corresponds to a situation, wherein the first quaternary electrode pair detects fluid, such as output, between the electrode assembly and the skin of the user in the first quaternary sensing zone. The first quaternary monitor signal may be indicative of a high risk of leakage or presence of output in the first quaternary sensing zone.

In one or more exemplary monitor devices, to determine an operating state of the base plate is based on a first quaternary criteria set based on the first quaternary parameter data, wherein the operating state is determined to be the first quaternary operating state if the first quaternary criteria set is satisfied.

The first quaternary criteria set may comprise or be given by

$$(P\_1\_4 < TH\_1\_4)$$

wherein $P\_1\_4$ is a first quaternary parameter based on the first quaternary parameter data and indicative of the resistance between electrodes of the first quaternary electrode pair, $TH\_1\_4$ is a first quaternary threshold value, and the first quaternary operating state is indicative of high risk of leakage or presence of output in the first quaternary sensing zone of the electrode assembly/base plate/ostomy appliance.

To apply a processing scheme may comprise to obtain first quinary parameter data based on first quinary ostomy data (from first quinary electrode pair) of the ostomy data and determine an operating state of the base plate of the ostomy appliance based on the first quinary parameter data. The monitor device may be configured to, in accordance with a determination that the operating state is a first quinary operating state, transmit a first quinary monitor signal comprising monitor data indicative of the first quinary operating state of the ostomy appliance.

In one or more exemplary monitor devices, the first quinary operating state of the base plate corresponds to a situation, wherein the first quinary electrode pair detects fluid, such as output, between the electrode assembly and the skin of the user in the first quinary sensing zone. The first quinary monitor signal may be indicative of a high risk of leakage or presence of output in the first quinary sensing zone.

In one or more exemplary monitor devices, to determine an operating state of the base plate is based on a first quinary criteria set based on the first quinary parameter data, wherein the operating state is determined to be the first quinary operating state if the first quinary criteria set is satisfied.

The first quinary criteria set may comprise or be given by

$$(P\_1\_5 < TH\_1\_5)$$

wherein $P\_1\_5$ is a first quinary parameter based on the first quinary parameter data and indicative of the resistance between electrodes of the first quinary electrode pair, $TH\_1\_5$ is a first quinary threshold value, and the first quinary operating state is indicative of high risk of leakage or presence of output in the first quinary sensing zone of the electrode assembly/base plate/ostomy appliance.

To apply a processing scheme may comprise to obtain first senary parameter data based on first senary ostomy data (from first senary electrode pair) of the ostomy data, and determine an operating state of the base plate of the ostomy appliance based on the first senary parameter data. The monitor device may be configured to, in accordance with a determination that the operating state is a first senary operating state, transmit a first senary monitor signal comprising monitor data indicative of the first senary operating state of the ostomy appliance.

In one or more exemplary monitor devices, the first senary operating state of the base plate corresponds to a situation, wherein the first senary electrode pair detects fluid, such as output, between the electrode assembly and the skin of the user in the first senary sensing zone. The first senary monitor signal may be indicative of a high risk of leakage or presence of output in the first senary sensing zone.

In one or more exemplary monitor devices, to determine an operating state of the base plate is based on a first senary criteria set based on the first senary parameter data, wherein the operating state is determined to be the first senary operating state if the first senary criteria set is satisfied.

The first senary criteria set may comprise or be given by

$$(P\_1\_6 < TH\_1\_6)$$

wherein $P\_1\_6$ is a first senary parameter based on the first senary parameter data and indicative of the resistance between electrodes of the first senary electrode pair, $TH\_1\_6$ is a first senary threshold value, and the first senary operating state is indicative of high risk of leakage or presence of output in the first senary sensing zone of the electrode assembly/base plate/ostomy appliance.

In one or more exemplary monitor devices, the ostomy data comprises second primary ostomy data from a second primary electrode pair (e.g. second primary electrode and first primary reference electrode) of the electrode assembly. To apply a processing scheme may comprise to obtain second primary parameter data based on the second primary ostomy data and determine an operating state of the base plate of the ostomy appliance based on the second primary parameter data. The monitor device may be configured to, in accordance with a determination that the operating state is a second primary operating state, transmit a second primary monitor signal comprising monitor data indicative of the second primary operating state of the ostomy appliance or the base plate of the ostomy appliance.

One or more of the first criteria sets, such as the first primary criteria set, the first secondary criteria set, and the first tertiary criteria set, may comprise

$$(P\_2\_1 < TH\_2\_1)$$

wherein $P\_2\_1$ is a second primary parameter based on the second primary parameter data and indicative of the resistance between the second primary electrode pair, and $TH\_2\_1$ is a second primary threshold value. Thus, the respective first operating states may be indicative of a medium risk of leakage or presence of output in the respective first sensing zones (but not in the second primary sensing zone).

In one or more exemplary monitor devices, the second primary operating state of the electrode assembly corresponds to a situation, wherein the second primary electrode pair detects fluid, such as output, between the electrode assembly and the skin of the user at a second primary distance, and thus there is a high risk of leakage from the ostomy appliance in the second primary operating state.

In one or more exemplary monitor devices, to determine an operating state of the base plate is based on a second primary criteria set based on the second primary parameter data, wherein the operating state is determined to be the second primary operating state if the second primary criteria set is satisfied.

The second primary criteria set may comprise or be given by

$$(P\_2\_1 < TH\_2\_1)$$

wherein $P\_2\_1$ is a second primary parameter based on the second primary parameter data and indicative of the resistance between the second primary electrode pair, and $TH\_2\_1$ is a second primary threshold value, and wherein the second primary operating state is indicative of high risk of leakage from the ostomy appliance. In other words, the second primary operating state may indicative of a high risk of leakage or presence of output in the second primary sensing zone.

The monitor device comprises a monitor device housing optionally made of a plastic material. The monitor device housing may be an elongate housing having a first end and a second end. The monitor device housing may have a length or maximum extension along a longitudinal axis in the range from 1 cm to 15 cm. The monitor device housing may have a width or maximum extension perpendicular to the longitudinal axis in the range from 0.5 cm to 3 cm. The monitor device housing may be curve-shaped.

The monitor device comprises a first interface. The first interface may be configured as an appliance interface for electrically and/or mechanically connecting the monitor device to the electrode assembly/ostomy appliance. Thus, the appliance interface is configured to electrically and/or mechanically couple the monitor device and the electrode assembly ostomy appliance. The first interface may be configured as an accessory device interface for electrically and//or mechanically connecting the monitor device to an accessory device, such as a docking station. The first interface may be configured for coupling to a docking station of the ostomy system, e.g. for charging the monitor device and/or for data transfer between the monitor device and the docking station.

The first interface of the monitor device may comprise a plurality of terminals, such as two, three, four, five, six, seven or more terminals, for forming electrical connections with respective terminals and/or electrodes of the electrode assembly/ostomy appliance. One or more terminals of the first interface may be configured for forming electrical connections with an accessory device, e.g. with respective terminals of a docking station. The first interface may comprise one or more reference terminals including a first primary reference terminal and/or a first secondary reference terminal. The first interface may comprise a first primary terminal and/or a second primary terminal. The first interface optionally comprises a first secondary terminal and/or a first tertiary terminal. The first interface may comprise a third primary terminal. In one or more exemplary monitor devices, the first interface has M terminals, wherein M is an integer in the range from 4 to 8.

The first interface of the monitor device may comprise a coupling part for forming a mechanical connection, such as a releasable coupling between the monitor device and the electrode assembly/base plate/ostomy appliance. The coupling part and the terminals of the first interface form (at least part of) a first connector of the monitor device.

The monitor device comprises a power unit for powering the monitor device. The power unit may comprise a battery. The power unit may comprise charging circuitry connected to the battery and terminals of the first interface for charging the battery via the first interface, e.g. the first connector. The first interface may comprise separate charging terminal(s) for charging the battery.

The monitor device may comprise a sensor unit with one or more sensor. The sensor unit is connected to the processor for feeding sensor data to the processor. The sensor unit may comprise an accelerometer for sensing acceleration and provision of acceleration data to the processor. The sensor unit may comprise a temperature sensor for provision of temperature data to the processor.

The monitor device comprises a second interface connected to the processor. The second interface may be configured as an accessory interface for connecting, e.g. wirelessly connecting, the monitor device to one or more accessory devices. The second interface may comprise an antenna and a wireless transceiver, e.g. configured for wireless communication at frequencies in the range from 2.4 to 2.5 GHz. The wireless transceiver may be a Bluetooth transceiver, i.e. the wireless transceiver may be configured for wireless communication according to Bluetooth protocol, e.g. Bluetooth Low Energy, Bluetooth 4.0, Bluetooth 5. The second interface optionally comprises a loudspeaker and/or a haptic feedback element for provision of an audio signal and/or haptic feedback to the user, respectively.

In one or more exemplary ostomy systems, the monitor device forms an integrated part of the ostomy appliance, e.g. the monitor device may form an integrated part of a base plate of the ostomy appliance. The monitor device may be electrically coupled to the plurality of electrodes of the base plate and/or the electrode assembly. For example, the monitor device may be couplable, such as releasably couplable, to the plurality of electrodes of the base plate and/or the electrode assembly. The monitor device may be configured to measure one or more resistances between the plurality of electrodes and detect the leakage of output based on the measured one or more resistances.

The ostomy system may comprise a docking station forming an accessory device of the ostomy system. The docking station may be configured to electrically and/or mechanically couple the monitor device to the docking station.

The docking station may comprise a docking monitor interface. The docking monitor interface may be configured for electrically and/or mechanically connecting the monitor device to the docking station. The docking monitor interface may be configured for wirelessly connecting the monitor device to the docking station. The docking monitor interface of the docking station may be configured to electrically and/or mechanically couple the docking station and the monitor device.

The docking monitor interface of the docking station may comprise, e.g. as part of a first connector of the docking monitor interface, a coupling part for forming a mechanical connection, such as a releasable coupling between the monitor device and the docking station. The coupling part may be configured to engage with a coupling part of the monitor device for releasably coupling the monitor device to the docking station.

The docking monitor interface of the docking station may comprise, e.g. as part of a first connector of the docking monitor interface, a plurality of terminals, such as two, three, four, five, six, seven or more terminals, for forming electrical connections with respective terminals of the monitor device. The docking monitor interface may comprise a ground terminal. The docking monitor interface may comprise a first terminal and/or a second terminal. The docking station may comprise a third terminal. The docking monitor interface may comprise a fourth terminal and/or a fifth terminal. The docking monitor interface optionally comprises a sixth terminal.

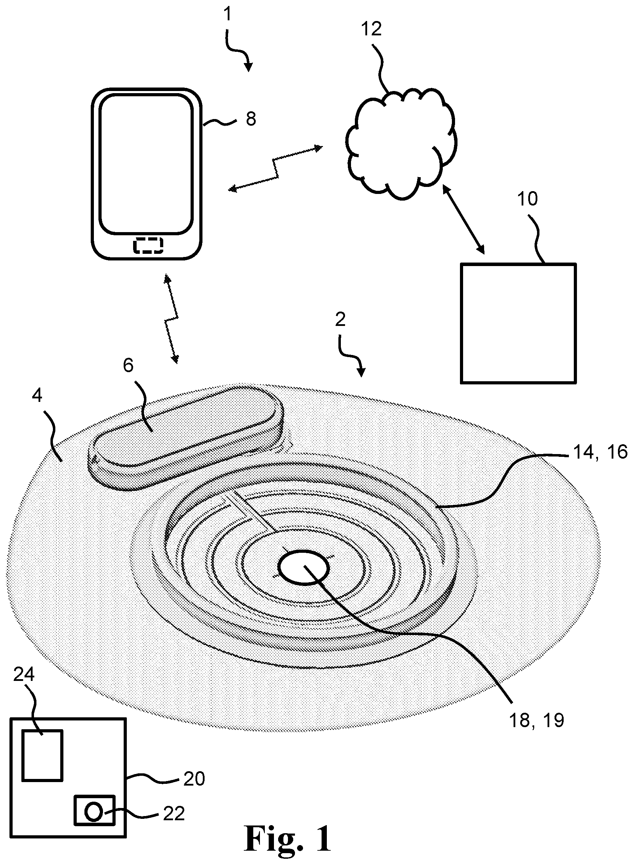

Fig. 1 illustrates an exemplary ostomy system. The ostomy system 1 comprises an ostomy appliance 2 including a base plate 4. The base plate 4 is adapted to support an ostomy pouch (not shown). Further, the ostomy system 1 comprises a monitor device 6 and an accessory device 8 (mobile telephone). The monitor device 6 is connectable to the base plate 4 via respective first connectors of the monitor device 6 and base plate 4. The monitor device 6 is configured for wireless communication with the accessory device 8. Optionally, the accessory device 8 is configured to communicate with a server device 10 of the ostomy system 1, e.g. via network 12. The server device 10 may be operated and/or controlled by the ostomy appliance manufacturer and/or a service centre. Ostomy data or parameter data based on the ostomy data are obtained from electrodes/sensors of the ostomy appliance 2 with the monitor device 6. The monitor device 6 processes the ostomy data and/or parameter data based on the ostomy data. Based on the processed ostomy data, the monitor device 6 may determine the monitor data that are transmitted to the accessory device 8. In the illustrated ostomy system, the accessory device 8 is a mobile phone, however the accessory device 8 may be embodied as another handheld device, such as a tablet device, or a wearable, such as a watch or other wrist-worn electronic device. Accordingly, the monitor device 6 is configured to determine and transmit monitor data to the accessory device 8. The base plate 4 comprises a coupling member 14 in the form of a coupling ring 16 for coupling an ostomy pouch (not shown) to the base plate (two-part ostomy appliance). The base plate 4 has a stomal opening 18 with a centre point 19. The size and/or shape of the stomal opening 18 is typically adjusted by the user or nurse before application of the ostomy appliance to accommodate the user's stoma.

The ostomy system 1 optionally comprises a docking station 20 forming an accessory device of the ostomy system 1. The docking station 20 comprises a docking monitor interface including a first connector 22 configured for electrically and/or mechanically connecting the monitor device 6 to the docking station 20. The docking monitor interface may be configured for wirelessly connecting the monitor device to the docking station. The docking station 20 comprises a user interface 24 for receiving user input and/or providing feedback to the user on the operational state of the docking station 20. The user interface 24 may comprise a touch-screen. The user interface 24 may comprise one or more physical buttons and/or one or more visual indicators, such as light emitting diodes.

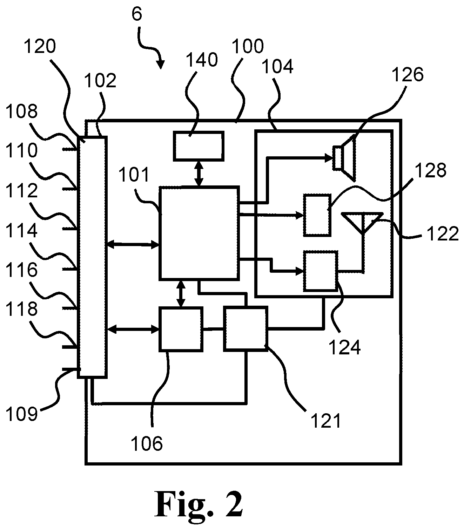

Fig. 2 is a schematic block diagram of an exemplary monitor device. The monitor device 6 comprises a monitor device housing 100, a processor 101, and one or more interfaces, the one or more interfaces including a first interface 102 (appliance interface) and a second interface 104 (accessory interface). The monitor device 6 comprises a memory 106 for storing ostomy data and/or parameter data based on the ostomy data. The memory 106 is connected to the processor 101 and/or the first interface 102.

The first interface 102 is configured as an appliance interface for electrically and/or mechanically connecting the monitor device 6 to the electrode assembly/ostomy appliance, e.g. ostomy appliance 2. The first interface 102 comprises a plurality of terminals for forming electrical connections with respective terminals of the ostomy appliance 2 (base plate 4). The first interface 102 comprises a first primary reference terminal 108, optionally a first secondary reference terminal 109, a first primary terminal 110, a first secondary terminal 112 and a first tertiary terminal 114. The first interface 102 optionally comprises a second primary terminal 116 and/or a third primary terminal 118. The first interface 102 of the monitor device 6 comprises a coupling part 120 for forming a mechanical connection, such as a releasable coupling between the monitor device and the electrode assembly/base plate/ostomy appliance. The coupling part 120 and the terminals 108, 109, 110, 112, 114, 116, and 118 of the first interface 102 form (at least part of) a first connector of the monitor device 6.

The monitor device 6 comprises a power unit 121 for powering the monitor device and active components thereof, i.e. the power unit 121 is connected to the processor 101, the first interface 102, the second interface 104, and memory 106. The power unit comprises a battery and charging circuitry. The charging circuitry is connected to the battery and terminals of the first interface 102 for charging the battery via terminals of the first interface, e.g. terminals of the first connector.

The second interface 104 of monitor device is configured as an accessory interface for connecting the monitor device 6 to one or more accessory devices such as accessory device 8. The second interface 104 comprises an antenna 122 and a wireless transceiver 124 configured for wireless communication with accessory device(s). Optionally, the second interface 104 comprises a loudspeaker 126 and/or a haptic feedback element 128 for provision of respective audio signal and/or haptic feedback to the user.

The monitor device 6 optionally comprises a sensor unit 140 connected to the processor 101. For example, the sensor unit 140 may comprise a temperature sensor for feeding temperature data to the processor and/or a G-sensor or accelerometer for feeding acceleration data to the processor 101. Additionally or alternatively, the sensor unit 140 comprises a humidity sensor and/or an acoustic sensor. The sensor unit 140 may comprise alternative and/or additional sensors suitable and/or relevant to an ostomy system as described.

The processor 101 is configured to apply a processing scheme as described herein, and the first interface 102 is configured for collecting ostomy data from the base plate coupled to the first interface, the ostomy data comprising one or more of first primary ostomy data, first secondary ostomy data, and first tertiary ostomy data from electrode pairs of the ostomy appliance. The electrode pairs are formed by electrodes of the electrode assembly connected to the terminals 108, 109, 110, 112, 114, 11, and 118. The ostomy data optionally comprises second primary ostomy data from a second primary electrode pair of the electrode assembly and/or the base plate and/or third primary ostomy data from a third primary electrode pair of the electrode assembly and/or the base plate. The ostomy data may comprise first reference ostomy data from a reference electrode of the electrode assembly and/or the base plate. The ostomy data may be stored in the memory 106 and/or processed in the processor 101 in order to obtain parameter data. The parameter data may be stored in the memory 106. The processor 101 is configured to apply a processing scheme, wherein to apply a processing scheme comprises to obtain first primary parameter data based on first primary ostomy data; obtain first secondary parameter data based on first secondary ostomy data; and obtain first tertiary parameter data based on first tertiary ostomy data. Optionally the processing scheme comprises to obtain first primary parameter data based on the first primary ostomy data; obtain first secondary parameter data based on the first secondary ostomy data; obtain first tertiary parameter data based on the first tertiary ostomy data. In other words, the processor 101 may be configured to obtain first primary parameter data, first secondary parameter data, and first tertiary parameter data based on respective first primary ostomy data, first secondary ostomy data, and first tertiary ostomy data. To apply a processing scheme comprises to determine an operating state of the base plate of the ostomy appliance based on one or more, e.g. all, of first primary parameter data, first secondary parameter data, and first tertiary parameter data. The operating state may be indicative of an acute leakage risk or presence of output in a sensing zone for the ostomy appliance. The monitor device 6 is optionally configured to, in accordance with a determination that the operating state is a first primary operating state, transmit a first primary monitor signal comprising monitor data indicative of the first primary operating state of the base plate via the second interface. The monitor device 6 is optionally configured to, in accordance with a determination that the operating state is a first secondary operating state, transmit a first secondary monitor signal comprising monitor data indicative of the first secondary operating state of the base plate via the second interface. The monitor device 6 may be configured to, in accordance with a determination that the operating state is a first tertiary operating state, transmit a first tertiary monitor signal comprising monitor data indicative of the first tertiary operating state of the base plate via the second interface. The monitor device 6 may be configured to, in accordance with a determination that the operating state is a first quaternary operating state, transmit a first quaternary monitor signal comprising monitor data indicative of the first quaternary operating state of the base plate via the second interface. The monitor device 6 may be configured to, in accordance with a determination that the operating state is a first quinary operating state, transmit a first quinary monitor signal comprising monitor data indicative of the first quinary operating state of the base plate via the second interface. The monitor device 6 may be configured to, in accordance with a determination that the operating state is a first senary operating state, transmit a first senary monitor signal comprising monitor data indicative of the first senary operating state of the base plate via the second interface. The monitor device 6 may be configured to, in accordance with a determination that the operating state is a default operating state, transmit a default monitor signal comprising monitor data indicative of the default operating state of the base plate via the second interface. The monitor device 6 may be configured to, in accordance with a determination that the operating state is a second primary operating state, transmit a second primary monitor signal comprising monitor data indicative of the second primary operating state of the base plate via the second interface.

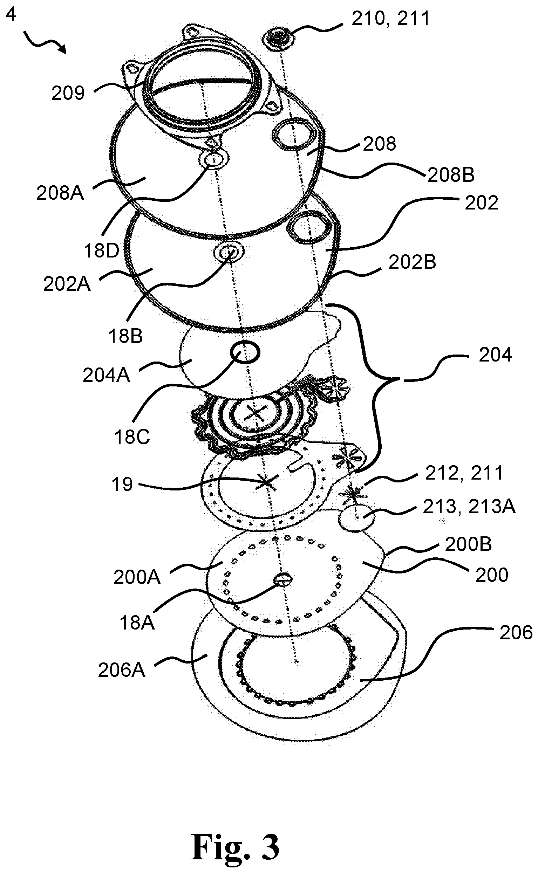

Fig. 3 illustrates an exploded view of an exemplary base plate of an ostomy appliance. The base plate 4 comprises a first adhesive layer 200 with a stomal opening 18A. During use, a proximal surface of the first adhesive layer 200 adheres to the user's skin in the peristomal area and/or to additional seals, such as sealing paste, sealing tape and/or sealing ring. The base plate 4 optionally comprises a second adhesive layer 202, also denoted rim adhesive layer, with a stomal opening 18B. The base plate 4 comprises a plurality of electrodes arranged in an electrode assembly 204. The electrode assembly 204 is arranged between the first adhesive layer 200 and the second adhesive layer 202. The electrode assembly 204 comprises a support layer with stomal opening 18C and electrodes formed on a proximal surface of the support layer. The base plate 4 comprises a base release liner 206 that is peeled off by the user prior to applying the base plate 4 on the skin. The base plate 4 comprises a top layer 208 with a stomal opening 18D and a coupling ring 209 for coupling an ostomy pouch to the base plate 4. The top layer 208 is a protective layer protecting the second adhesive layer 202 from external strains and stress during use.

The base plate 4 or electrode part 204 comprises a monitor interface. The monitor interface is configured for electrically and/or mechanically connecting the electrode assembly/ostomy appliance (base plate 4) to the monitor device. The monitor interface of the electrode assembly and/or base plate comprises a support member comprising coupling part 210 for forming a mechanical connection, such as a releasable coupling between the monitor device and the electrode assembly and/or base plate. The coupling part 210 is configured to engage with a coupling part of the monitor device for releasably coupling the monitor device to the electrode assembly and/or base plate 4. Further, the monitor interface of the electrode assembly and/or base plate 4 optionally comprises a plurality of terminal elements respectively forming a plurality of terminals 212 for forming electrical connections with respective terminals of the monitor device. The terminal elements are connected to respective connection parts of the electrodes. The coupling part 210 and the terminals 212 form a first connector 211 of the electrode assembly and/or base plate 4. The base plate 4 optionally comprises a first intermediate element 213 on the proximal side of the electrode assembly. The first intermediate element 213 may be arranged between the terminal elements forming terminals 212 and the first adhesive layer (not shown). The first intermediate element 213 covers the terminal elements forming terminals 212 of the base plate 4 when seen in the axial direction and protects the first adhesive layer from mechanical stress from the terminal elements of the base plate.

As previously described, some parts of the illustrated base plate 4, may be provided as a separate electrode assembly to be applied to an existing base plate, e.g. comprising one or more of the components as described, such as to provide a base plate like the base plate 4 as described. It may be envisioned that the user may provide a hole in layers of the base plate whereto the electrode assembly is to be applied, to allow for the first connector 211 of electrode assembly to protrude through layers of the base plate whereto the electrode assembly is applied. Alternatively, the electrode assembly may be applied to the base plate such that the first connector 211 is positioned outside the periphery of the base plate.

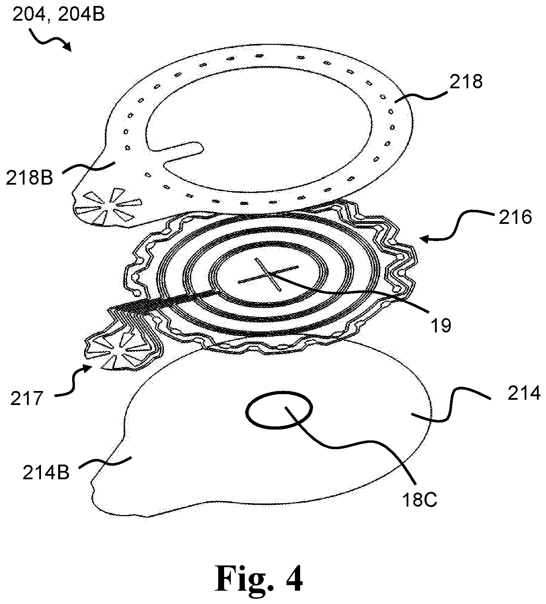

Fig. 4 illustrates an exploded view of an exemplary electrode assembly 204 of a base plate (integrated in the base plate and arranged on distal side of first adhesive layer). The electrode assembly 204 has a distal side 204A and a proximal side 204B. The electrode assembly 204 comprises a support layer 214 with proximal surface 214B and electrodes 216 arranged on the proximal side of the support layer 214 and including a first primary reference electrode, a first primary electrode, a first secondary electrode, a second primary electrode, a third primary electrode, and a fourth primary electrode, wherein each electrode has a respective connection part 217 for connecting the electrodes 216 to respective terminal elements of the monitor interface. The electrodes 216 are positioned and/or formed on a proximal side 214B of the support layer 214. Further, electrode assembly 204 comprises a masking layer or element 218 with proximal surface 218B and configured to insulate electrode parts of some of electrodes 216 from the first adhesive layer of the base plate to form conductor parts. The masking layer/element 218 covers or overlap with parts of the electrodes 216 when seen in the axial direction.

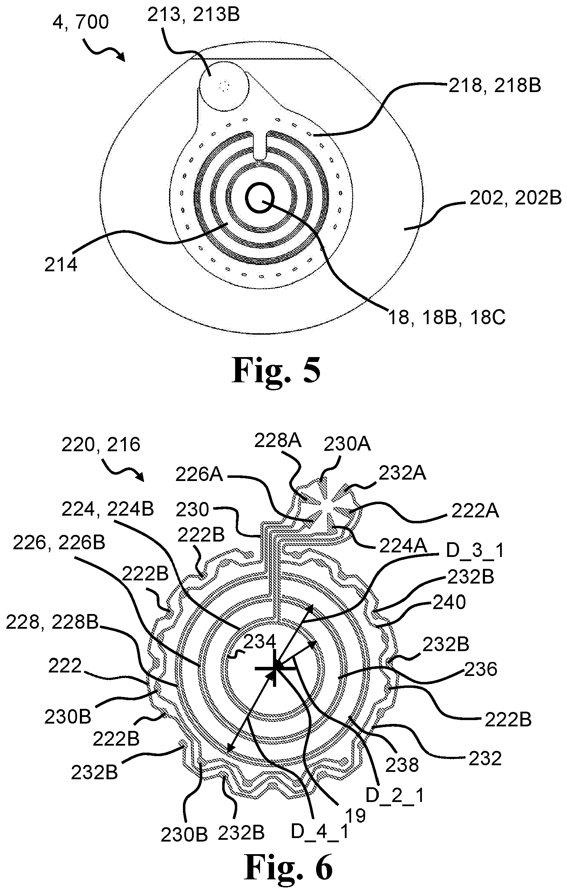

Fig. 5 is a proximal view of proximal surfaces of base plate parts of the base plate without the first adhesive layer and the base release liner. The base plate 4 optionally comprises a first intermediate element 213 on the proximal side of the electrode assembly, i.e. between the electrode assembly 204 and the first adhesive layer (not shown). The first intermediate element 213 covers the terminal elements of the base plate 4 when seen in the axial direction and protects the first adhesive layer from mechanical stress from the terminal elements of the base plate.

Fig. 6 is a distal view of an exemplary electrode configuration 220 of electrodes 216 of the electrode assembly 204. The electrode assembly 204, such as the electrode configuration 220 of the electrode assembly 204 comprises a first primary reference electrode 222, a second primary electrode 224, a third primary electrode 226, a fourth primary electrode 228, a first primary electrode 230, and a first secondary electrode 232. The first primary reference electrode 222 comprises a first primary reference connection part 222A and the second primary electrode 224 comprises a second primary connection part 224A. The third primary electrode 226 comprises a third primary connection part 226A, and the fourth primary electrode 228 comprises a fourth primary connection part 228A. The first primary electrode 230 comprises a first primary connection part 230A and the first secondary electrode 232 comprise a first secondary connection part 232A.

The first primary electrode 230 comprises sensing parts 230B. The first secondary electrode 232 comprises sensing parts 232B.

The first primary reference electrode 222 comprises a second electrode branch 234 for forming a ground or reference for the second primary electrode 224. The first primary reference electrode 222 optionally comprises a third electrode branch 236 for forming a ground or reference for the third primary electrode 226. The first primary reference electrode 222 optionally comprises a fourth electrode branch 238 for forming a ground or reference for the fourth primary electrode 228. The masking layer/element 218 is arranged proximal to the electrodes 222, 224, 226, 228, 230, 232 covering and insulating parts of the electrodes thereby forming respective conductor parts of the electrodes 222, 224, 226, 228. The parts of the electrodes 224, 226, 228, 230, 232 not covered by the masking element 218 are exposed via sensor point openings in first adhesive layer/proximal adhesive layer or contact the first adhesive layer/proximal adhesive layer and thereby form sensing parts of the electrodes 222, 224, 226, 228, 230, 232. Further, the electrode branches 234, 236, 238 form respective sensing parts of the first primary reference electrode 222.

The primary sensing part 224B of the second primary electrode 224 extends circularly at least 330 degrees around the stomal opening at a second primary distance D_2_1 from the centre point 19. The second electrode branch 234 is arranged on the inside of the primary sensing part 224B (i.e. closer to the centre point) and extends circularly at least 330 degrees around the stomal opening at a second ground distance RG2 from the primary sensing part 224B (radially from the centre point). The second ground distance RG2 is about 1 mm.

The primary sensing part 226B of the third primary electrode extends circularly at least 330 degrees around the stomal opening at a third primary distance D_3_1 from the centre point 19. The third electrode branch 236 is arranged on the inside of the primary sensing part 226B (i.e. closer to the centre point) and extends circularly at least 330 degrees around the stomal opening at a third ground distance RG3 from the primary sensing part 226B (radially from the centre point). The third ground distance RG3 is about 1 mm.

The primary sensing part 228B of the fourth primary electrode extends circularly at least 330 degrees around the stomal opening at a fourth primary distance D_4_1 from the centre point 19. The fourth electrode branch 238 is arranged on the inside of the primary sensing part 228B (i.e. closer to the centre point) and extends circularly at least 330 degrees around the stomal opening at a third ground distance RG3 from the primary sensing part 228B (radially from the centre point). The third ground distance RG3 is about 1 mm.

The first primary reference electrode 222 comprises a first electrode branch 240 comprising a plurality of sensing parts for forming a ground or reference for the first primary electrode 230 and the first secondary electrode 232. The first electrode branch 240 of the first primary reference electrode 222 extends at least 300 degrees around the stomal opening and comprises sensing parts 222B. The sensing parts 230B of the first primary electrode, sensing parts 232B of the first secondary electrode, and sensing parts 222B of the first electrode branch 240 are circularly distributed around the centre point 19 at first distance from the centre point 19. The sensing parts 230B, sensing parts 232B, and sensing parts 222B of the first electrode branch may each have a radial extension larger than 1.0 mm, such as in the range from 1.5 mm to 3.0 mm, e.g. about 2.0 mm. The sensing parts 230B, sensing parts 232B, and sensing parts 222B of the first electrode branch 240 may have a circumferential extension (perpendicular to the radial extension) larger than 1.0 mm, such as in the range from 2.5 mm to 5.0 mm, e.g. about 3.5 mm. In one or more exemplary electrode assemblies and/or base plates, one or more or the electrodes 224, 226, 228 and corresponding electrode branches 234, 236, 238 may be omitted from the electrode configuration/electrode assembly.

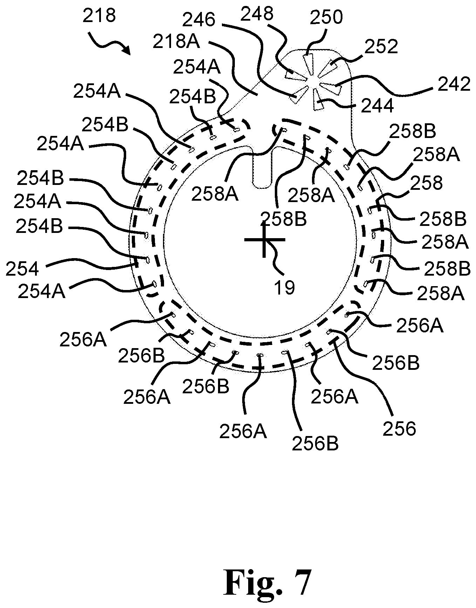

Fig. 7 is a distal view of an exemplary masking element e.g. for electrode configuration 220 illustrated in Fig. 6. The masking element or layer 218 optionally has a plurality of terminal openings including six terminal openings. The terminal openings 242, 244, 246, 248, 250, 252 of the masking element 218 are configured to overlap and/or be aligned with respective connection parts 222A, 224A, 226A, 228A, 230A, 232A of the electrodes of the electrode assembly.

The masking element 218 has a plurality of sensor point openings. The sensor point openings comprise first primary sensor point openings shown within dotted line 254, each first primary sensor point opening configured to overlap a sensing part of the first primary reference electrode 222 and/or a sensing part of the first primary electrode 230. The first primary sensor point openings 254 comprise, in the illustrated exemplary masking element, five first primary sensor point openings 254A each configured to overlap a respective primary sensing part of the first primary reference electrode 222. The first primary sensor point openings 254 comprise, in the illustrated exemplary masking element/layer, four first primary sensor point openings 254B each configured to overlap a respective primary sensing part of the first primary electrode 230. The plurality of sensor point openings comprise first secondary sensor point openings shown within dotted line 256, each first secondary sensor point opening configured to overlap a part of the first primary electrode 230 and/or a part of the first secondary electrode 232. The first secondary sensor point openings 256 comprise, in the illustrated exemplary masking element, five first secondary sensor point openings 256A each configured to overlap a respective secondary sensing part of the first secondary electrode 232. The first secondary sensor point openings 256 comprise, in the illustrated exemplary masking element, four first secondary sensor point openings 256B each configured to overlap a respective secondary sensing part of the first primary electrode 230. The sensor point openings comprise first tertiary sensor point openings shown within dotted line 258, each first tertiary sensor opening configured to overlap a part of the first secondary electrode 232 and/or a part of the first primary reference 222. The first tertiary sensor point openings 258 comprise, in the illustrated exemplary masking element, five first tertiary sensor point openings 258A each configured to overlap a respective tertiary sensing part of the first secondary electrode 232. The first tertiary sensor point openings 258 comprise, in the illustrated exemplary masking element, four first tertiary sensor point openings 258B each configured to overlap a respective tertiary sensing part of the first primary reference electrode (first electrode branch) 222. The sensor point openings 254A, 254B, 256A, 256B, 258A, 258B, are circularly arranged at a first distance of about 30 mm from the centre point 19, i.e. $D\_1\_1 = D\_1\_2 = D\_1\_3 = 30$ mm.

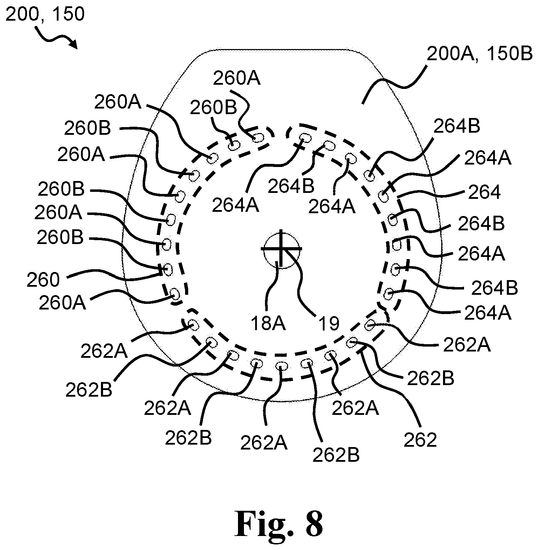

Fig. 8 is a distal view of an exemplary first adhesive layer 200 or proximal adhesive layer 150. The adhesive layer 150, 200 has a plurality of sensor point openings. The sensor point openings of the adhesive layer 150, 200 comprise first primary sensor point openings shown within dotted line 260, each first primary sensor point opening configured to overlap a part of the first primary reference electrode 222 and/or a part of the first primary electrode 230 of the electrode assembly. The first primary sensor point openings 260 comprise, in the illustrated exemplary adhesive layer 150, 200, five first primary sensor point openings 260A each configured to overlap a respective primary sensing part of the first primary reference electrode 222. The first primary sensor point openings 260 comprise, in the illustrated exemplary adhesive layer, four first primary sensor point openings 260B each configured to overlap a respective primary sensing part of the first primary electrode 230. The sensor point openings of the adhesive layer 150, 200 comprise first secondary sensor point openings shown within dotted line 262, each first second sensor point opening configured to overlap a part of the first primary electrode 230 and/or a part of the first secondary electrode 232 of the electrode assembly. The first secondary sensor point openings comprise, in the illustrated exemplary adhesive layer, five first secondary sensor point openings 262A each configured to overlap a respective secondary sensing part of the first secondary electrode 232. The first secondary sensor point openings comprise, in the illustrated exemplary adhesive layer, four first secondary sensor point openings 262B each configured to overlap a respective secondary sensing part of the first primary electrode 230. The sensor point openings of the first adhesive layer comprise first tertiary sensor point openings shown within dotted line 264, each first tertiary sensor opening configured to overlap a part of the first secondary electrode 232 and/or a part of the first primary reference electrode 222 of the electrode assembly. The first tertiary sensor point openings comprise, in the illustrated exemplary adhesive layer, five first tertiary sensor point openings 264A each configured to overlap a respective tertiary sensing part of the first secondary electrode 232. The first tertiary sensor point openings comprise, in the illustrated exemplary adhesive layer, four first tertiary sensor point openings 264B each configured to overlap a respective tertiary sensing part of the first primary reference electrode 222.

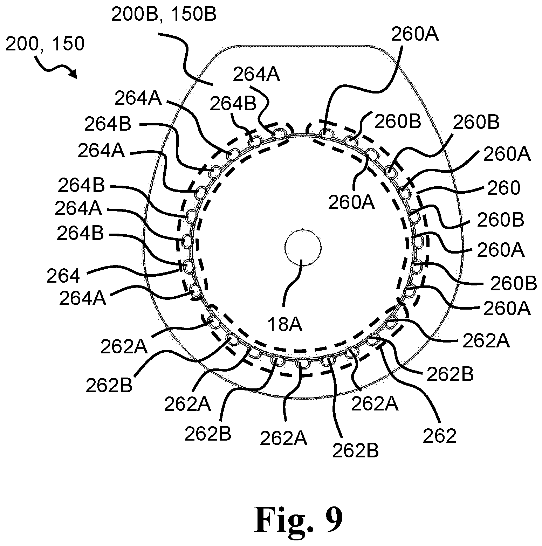

Fig. 9 is a proximal view of the first adhesive layer of Fig. 8. The sensor point openings 260A, 260B, 262A, 262B, 264A, 264B, are circularly arranged at a first distance of about 30 mm from the centre point 19, i.e. $D\_1\_1 = D\_1\_2 = D\_1\_3 = 30$ mm.

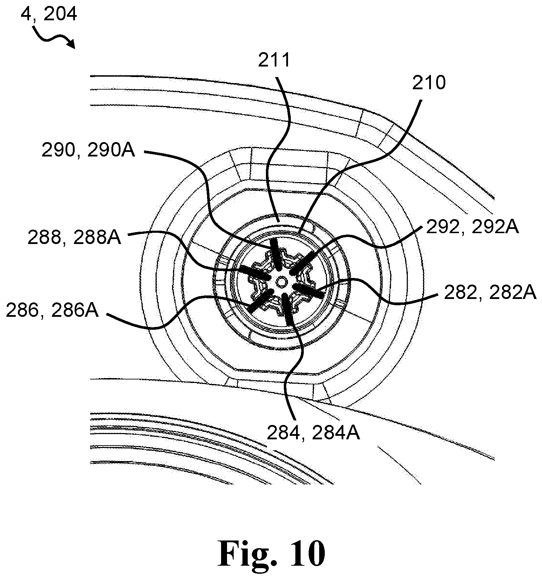

Fig. 10 is a more detailed distal view of a part of an exemplary electrode assembly 204 and/or base plate 4. Monitor interface of the electrode assembly and/or the base plate comprises the first connector 211. The first connector 211 comprises coupling part 210 configured to releasably couple the monitor device to the electrode assembly and/or base plate and thus forming a releasable coupling. The first connector 211/monitor interface comprises a plurality of terminals formed by respective terminal elements for forming respective electrical connections with respective terminals of the monitor device.

The plurality of terminals of the first connector 211/monitor interface comprises a ground terminal element 282 forming a ground terminal 282A, a first terminal element 284 forming a first terminal 284, a second terminal element 286 forming a second terminal 286A, and optionally a third terminal element 288 forming a third terminal 288A. The monitor interface optionally comprises a fourth terminal element 290 forming a fourth terminal 290A and/or a fifth terminal element 292 forming a fifth terminal 290. The terminal elements 282, 284, 286, 288, 290, 292 contact respective connection parts 222A, 224A, 226A, 228A, 230a, 232A of electrodes 222, 224, 226, 228, 230, 232.

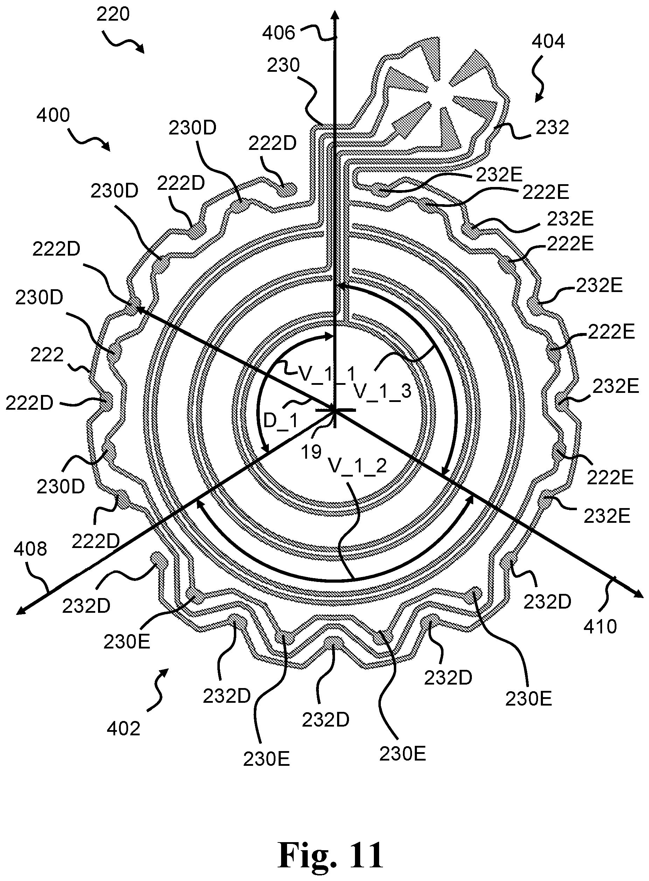

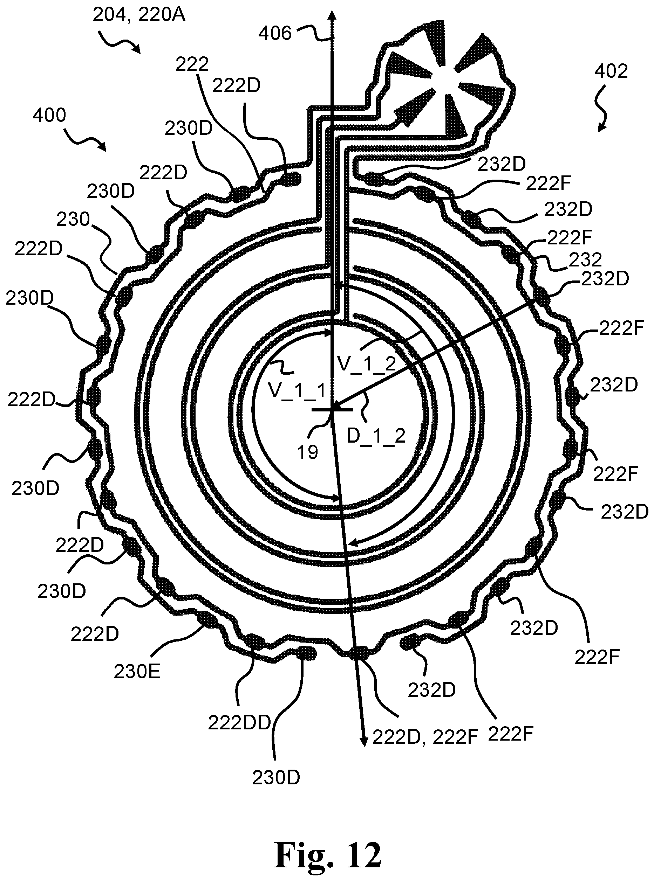

The position of the first connector on the base plate, the number of terminals and the position of the terminals in the coupling part may be adapted to the electrode configuration used in the electrode assembly of the base plate. For example, a first connector for a base plate with the electrode configuration 220 shown in Fig. 11 comprises four terminals respectively connected to connection parts 222A, 224A, 226A, 228A of the electrodes, and a first connector for a base plate with the electrode configuration 220A shown in Fig. 12 comprises three terminals respectively connected to connection parts 222A, 224A, 226A of the electrodes.

Fig. 11 is a distal view of the exemplary electrode configuration 220 of Fig. 6 for an electrode assembly and/or base plate. The base plate/ electrode configuration 220 comprises a first primary reference electrode 222, first primary electrode 230, and first secondary electrode 232. The electrodes 222, 230, 232 are configured to detect presence of fluid on the proximal side of the first adhesive layer or proximal adhesive layer in three angular sensing zones, first primary sensing zone 400, first secondary sensing zone 402, and first tertiary sensing zone 404. The first primary sensing zone 400 is arranged in a first primary angle space between a first direction 406 and a second direction 408 from the centre point 19, wherein the first primary angle space spans a first primary angle V_1_1 of 120°. The first secondary sensing zone 402 is arranged in a first secondary angle space between the second direction 408 and a third direction 410 from the centre point 19, wherein the first secondary angle space spans a first secondary angle V_1_2 of 120°. The first tertiary sensing zone 404 is arranged in a first tertiary angle space between the third direction 410 and the first direction 406 from the centre point 19, wherein the first tertiary angle space spans a first tertiary angle V_1_3 of 120°.

The first primary reference electrode 222 comprises five primary sensing parts 222D arranged in the primary sensing zone 400, and four tertiary sensing parts 222E arranged in the first tertiary sensing zone 404. Each primary sensing part 222D is aligned with a respective first primary sensor point opening 254A of the masking element 218 (see Fig. 7). Further, each primary sensing part 222D is aligned with a respective first primary sensor point opening 260A of the adhesive layer 150, 200 (see Fig. 8). Each tertiary sensing part 222E of the first primary reference electrode 222 is aligned with a respective first tertiary sensor point opening 258B of the masking element 218 (see Fig. 7). Further, each tertiary first sensing part 222E is aligned with a respective first tertiary sensor point opening 264B of the adhesive layer 150, 200 (see Fig. 8).

The first primary electrode 230 comprises four primary sensing parts 230D arranged in the first primary sensing zone 400, and four secondary sensing parts 230E arranged in the first secondary sensing zone 402. Each primary sensing part 230D is aligned with a respective first primary sensor point opening 254B of the masking element 218 (see Fig. 7). Further, each primary sensing part 230D is aligned with a respective first primary sensor point opening 260B of the adhesive layer 150, 200 (see Fig. 8). Each secondary sensing part 230E is aligned with a respective first secondary sensor point opening 256B of the masking element 218 (see Fig. 7). Further, each secondary sensing part 230E is aligned with a respective first secondary sensor point opening 262B of the adhesive layer 150, 200 (see Fig. 8).

The first secondary electrode 232 comprises five secondary sensing parts 232D arranged in the first secondary sensing zone 402, and five tertiary sensing parts 232E arranged in the first tertiary sensing zone 404. Each secondary sensing part 232D is aligned with a respective first secondary sensor point opening 256A of the masking element 218 (see Fig. 7). Further, each secondary sensing part 232D is aligned with a respective first secondary sensor point opening 262A of the adhesive layer 150, 200 (see Fig. 8). Each tertiary sensing part 232E is aligned with a respective first tertiary sensor point opening 258A of the masking element 218 (see Fig. 7). Further, each tertiary sensing part 232E is aligned with a respective first tertiary sensor point opening 264A of the adhesive layer 150, 200 (see Fig. 8). The sensing parts 222D, 222E, 230D, 230E, 232D, 232E are circularly arranged at a first distance D_1 of about 30 mm from the centre point 19, i.e. D_1_1 =D_1_2=D_1_3=30 mm.

Fig. 12 is a distal view of parts of an exemplary electrode assembly with electrode configuration 220A for an electrode assembly/base plate. The electrode configuration 220 comprises a first primary reference electrode 222, a first primary electrode 230, and first secondary electrode 232. The electrodes 222, 230, 232 are configured to detect presence of fluid on the proximal side of the first adhesive layer or proximal adhesive layer in two angular sensing zones, first primary sensing zone 400 and first secondary sensing zone 402. The first primary sensing zone 400 is arranged in a first primary angle space between a first direction 406 and a second direction 408 from the centre point 19, wherein the first primary angle space spans a first primary angle V_1_1 of about 185°. The first secondary sensing zone 402 is arranged in a first secondary angle space between the second direction 408 and the first direction 406 from the centre point 19, wherein the first secondary angle space spans a first secondary angle V_1_2 of about 175°.

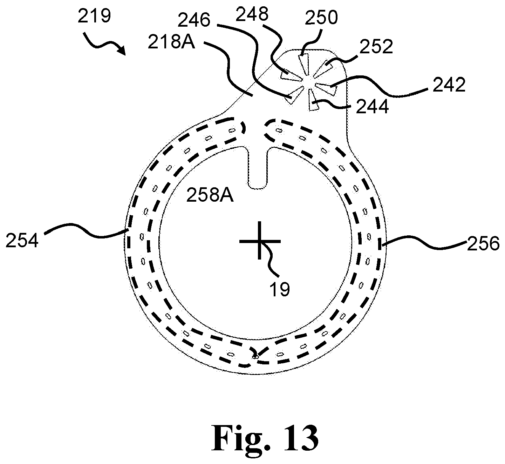

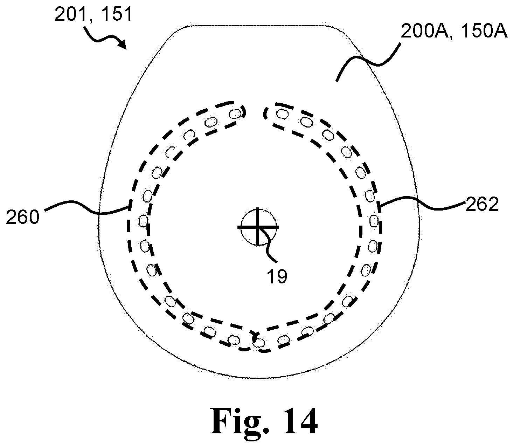

The first primary reference electrode 222 comprises primary sensing parts 222D arranged in the first primary sensing zone 400, and secondary sensing parts 222F arranged in the first secondary sensing zone 402. The first primary electrode 230 comprises primary sensing parts 230D arranged in the first primary sensing zone 400. The first secondary electrode 232 comprises secondary sensing parts 232D arranged in the first secondary sensing zone 402. Each primary sensing part 222D, 230D is aligned with a respective first primary sensor point opening of the masking element 219 (see Fig. 13) and with a respective first primary sensor point opening of an adhesive layer 151, 201 (see Fig. 14). The sensing parts 222D, 222F, 230D, and 232D are circularly arranged at a first distance of about 30 mm from the centre point 19, i.e. D_1_1 =D_1_2=D_1_3=30 mm.

Fig. 13 is a distal view of masking layer 219 for electrode configuration 220A in Fig. 12. The masking layer 219 comprises first primary sensor point openings 254 and first secondary sensor point openings 256. Fig. 14 is a distal view of first adhesive layer 201 or proximal adhesive layer 151 for electrode configuration 220A in Fig. 12 implementing a base plate with two sensing zones arranged in separate angle spaces. The adhesive layer 151, 201 comprises first primary sensor point openings 260 and first secondary sensor point openings 262.

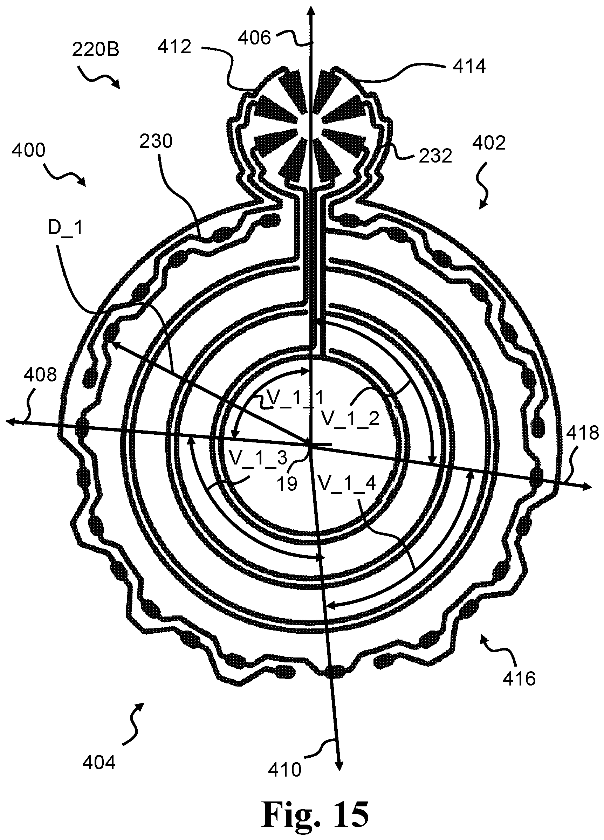

Fig. 15 is a distal view of an exemplary electrode configuration 220B for a base plate. The base plate/electrode configuration 220B comprises first primary reference electrode 222, first primary electrode 230, first secondary leakage electrode 232, first tertiary electrode 412, and optional first quaternary electrode 414. The electrodes 222, 230, 232, 412, 414 are configured to detect presence of fluid on the proximal side of the first adhesive layer or proximal adhesive layer in four angular sensing zones 400, 402, 404, 416. The first primary sensing zone 400 is arranged in a primary angle space spanning a primary angle V_1_1 of about 85°. The first secondary sensing zone 402 is arranged in a first secondary angle space spanning a first secondary angle V_1_2 of about 95°. The first tertiary sensing zone 404 is arranged in a first tertiary angle space spanning a first tertiary angle V_1_3 of about 95°. Optional first quaternary sensing zone 416 is arranged in a first quaternary angle space spanning a quaternary angle V_1_4 of about 85°. First sensing zones 400, 402, 404, 416 are arranged at first distance D_1 from the centre point 19.

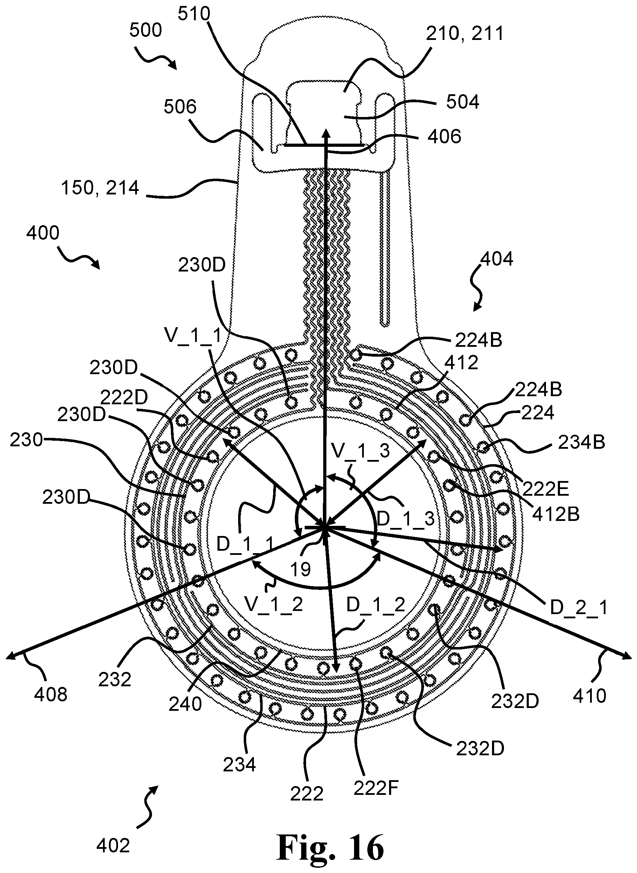

Fig. 16 is a distal view of an exemplary electrode assembly. The electrode assembly 500 comprises a support layer 214, the support layer having a stomal opening 18C with a centre point 19. The electrode assembly 500 comprises a plurality of electrodes printed on the proximal surface of the support layer and including a first set of electrodes, the first set of electrodes comprising one or more first electrodes including a first primary electrode 230, a first secondary electrode 232, and a first tertiary electrode 412. The plurality of electrodes includes one or more first reference electrodes including a first primary reference electrode 222. The electrodes are configured to detect presence of fluid in a first primary sensing zone 400, a first secondary sensing zone 402, and a first tertiary sensing zone 404. The first primary sensing zone 400 is arranged in a first primary angle space V_1_1 from the centre point of the support layer 214 and at a first primary distance D_1_1 from the centre point of the support layer. The first primary angle space V_1_1 spans a first primary angle in the range from 90° to 135° and the first primary distance D_1_1 is in the range from 20 mm to 45 mm. The first secondary sensing zone 402 is arranged in a first secondary angle space V_1_2 from the centre point 19 and at a first secondary distance D_1_2 from the centre point of the support layer. The first secondary angle space V_1_2 spans a first secondary angle in the range from 90° to 135° and the first secondary distance D_1_2 is in the range from 20 mm to 45 mm. The first tertiary sensing zone 404 is arranged in a first tertiary angle space V_1_3 from the centre point of the support layer and at a first tertiary distance D_1_3, from the centre point of the support layer. The first tertiary angle space V_1_3 spans a first tertiary angle in the range from 90° to 135° and the first tertiary distance D_1_3 is in the range from 20 mm to 45 mm. The electrode assembly 500 comprises a second primary electrode 224 with seventeen primary sensing parts 224B arranged in a second primary angle space larger than 300° at second primary distance D_2_1 in the range from 30-55 mm from the centre point 19. A second electrode branch 234 of the first primary reference electrode comprises seventeen primary sensing parts 234B arranged in the second primary angle space at second primary distance D_2_1 from the centre point 19. The second primary electrode 224 and the second electrode branch 234 of the first primary reference electrode (primary sensing parts 224B, 234B) form second primary electrode pair.

The first primary electrode 230 comprises a plurality of primary sensing parts 230D arranged in the first primary sensing zone 400. A first electrode branch 240 of the first primary reference electrode 222 comprises primary sensing parts 222D arranged in the first primary angle space V_1_1 at first primary distance D_1_1 from the centre point 19. The first primary electrode 230 and the first electrode branch 240 of the first primary reference electrode (primary sensing parts 222D, 230D) form first primary electrode pair.

The first secondary electrode 232 comprises a plurality of secondary sensing parts 232D arranged in the first secondary sensing zone 402. A first electrode branch 240 of the first primary reference electrode 222 comprises secondary sensing parts 222F arranged in the first secondary angle space V_1_2 at first secondary distance D_1_2 from the centre point 19. The first secondary electrode 232 and the first electrode branch 240 of the first primary reference electrode (secondary sensing parts 222F, 232D) form first secondary primary electrode pair.

The first tertiary electrode 412 comprises a plurality of tertiary sensing parts 412B arranged in the first tertiary sensing zone 404. A first electrode branch 240 of the first primary reference electrode 222 comprises tertiary sensing parts 222E arranged in the first tertiary angle space V_1_3 at first tertiary distance D_1_3 from the centre point 19. The first tertiary electrode 412 and the first electrode branch 240 of the first primary reference electrode (tertiary sensing parts 222E, 412B) form first tertiary electrode pair.

Sensor point openings of a masking layer and proximal adhesive layer overlap the sensing parts 222D, 230D, 222F, 232D, 412B, 222E, 224B, 234B. In other words, a sensor point opening of masking layer (not shown) overlaps each of the sensing parts 222D, 230D, 222F, 232D, 412B, 222E, 224B, 234B and a sensor point opening of the proximal adhesive layer overlaps sensing parts 222D, 230D, 222F, 232D, 412B, 222E, 224B, 234B.

The electrode assembly 500 comprises a support member 504 comprising a base part 506 and a coupling part 210 tiltably attached to the base part 506 via a joint formed by weakened line 510. Connection parts of electrodes 222, 224, 230, 232, 412 are arranged on proximal surface (not shown) of the coupling part 210 and form terminals of first connector 211. It is to be understood that the support member 504 may be embedded in any of the electrode assemblies disclosed herein.

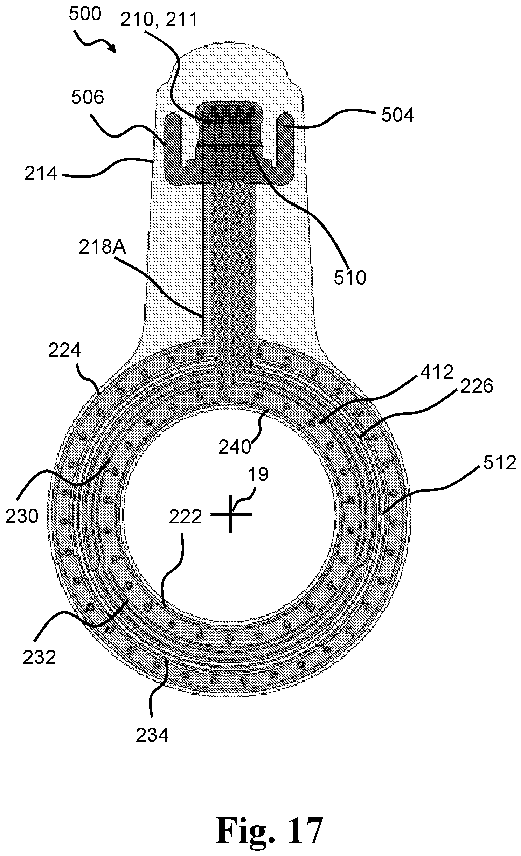

Fig. 17 is a proximal view of an exemplary electrode assembly, such as electrode assembly 500. The electrode assembly 500 optionally comprises proximal adhesive layer (not shown) or may be arranged on the distal side of a first adhesive layer of a base plate. The electrode assembly comprises a support member 504 with a base part 506 and a coupling part 210. A masking layer 218A is arranged on the proximal side of the support layer covering parts of electrode while sensor point openings of the masking layer 218A expose sensing parts of the electrodes either to contact an adhesive layer and/or to expose sensing parts of the electrodes through sensor point openings of the (first or proximal) adhesive layer. The masking layer 218A optionally comprises a third primary sensor point opening 512 covering a part of third primary electrode 226 and a part of the second electrode branch 234 to form a third primary sensing zone in a third primary angular space, e.g. larger than 300°, at a third primary distance from the centre 19. Thus, the second electrode branch 234 and the third primary electrode 226 may form a third primary electrode pair.

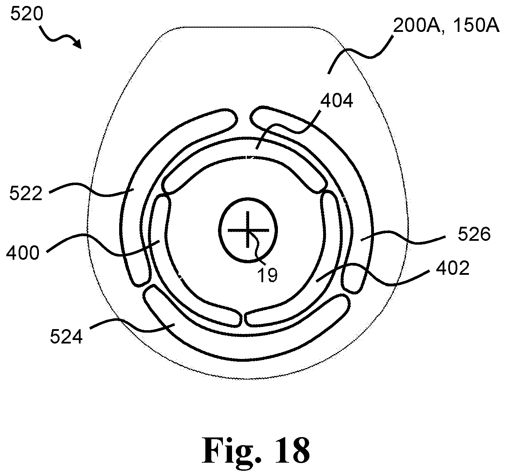

Fig. 18 schematically illustrates an exemplary sensing zone configuration 520 of an electrode assembly/base plate of the present disclosure. The plurality of electrodes of the electrode assembly is configured to detect presence of fluid in a second primary sensing zone 522, a second secondary sensing zone 524, and a second tertiary sensing zone 526. The second primary sensing zone 522 is arranged in a second primary angle space from the centre point of the support layer and at a second primary distance from the centre point of the support layer. The second secondary sensing 524 zone is arranged in a second secondary angle space from the centre point of the support layer and a second secondary distance from the centre point of the support layer. The second tertiary sensing zone 526 is arranged in a second tertiary angle space from the centre point of the support layer and at a second tertiary distance from the centre point of the support layer.

The second primary angle space of second primary sensing zone 522 partly overlaps the first primary angle space of first primary sensing part 400 with about 60°, i.e. the first primary angle space and the second primary angle space are offset by a primary offset angle of 60°. The second primary angle space of second primary sensing part 522 partly overlaps the first tertiary angle space of first tertiary sensing part 404 with about 60°, i.e. the first tertiary angle space and the second primary angle space are offset by 60°.

The second secondary angle space of second secondary sensing zone 524 partly overlaps the first primary angle space of first primary sensing part 400 with about 60°. The second secondary angle space of second secondary sensing part 524 partly overlaps the first secondary angle space of first secondary sensing part 402 with about 60°.

The second tertiary angle space of second tertiary sensing zone 526 partly overlaps the first secondary angle space of first secondary sensing part 402 with about 60°. The second tertiary angle space of second tertiary sensing part 526 partly overlaps the first tertiary angle space of first tertiary sensing part 404 with about 60°.

The sensing zone configuration 520 increases (doubles) the angular sensitivity of the leakage detection in a way which is easy to manufacture and which provides a relatively simple sensor layout without six parallel electrodes in immediate vicinity.

While exemplary base plates/electrode assemblies with two, three and four angular sensing zones have been described in more detail, the base plate/electrode assembly may comprise one or a larger number of sensing zones, such as five, six, seven, eight or more sensing zones.

The use of the terms "first", "second", "third" and "fourth", "primary", "secondary", "tertiary", "quaternary", "quinary", "senary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

Although particular features have been shown and described, it will be understood that they are not intended to limit the claimed invention, and it will be made obvious to those skilled in the art that various changes and modifications may be made without departing from the scope of the claimed invention. The specification and drawings are accordingly to be regarded in an illustrative rather than restrictive sense.

LIST OF REFERENCES

| 1 | ostomy system |
|---|---|
| 2 | ostomy appliance |
| 4 | base plate |
| 6 | monitor device |
| 8 | accessory device |
| 10 | server device |
| 12 | network |
| 14 | coupling member |
| 16 | coupling ring |
| 18, 18A, 18B, 18C, 18D | stomal opening |
| 19 | centre point |
| 20 | docking station |

| | |
|---|---|
| 22 | first connector |
| 24 | user interface |
| 100 | monitor device housing |
| 101 | processor |
| 102 | first interface |
| 104 | second interface |
| 106 | memory |
| 108 | first primary referenc terminal of monitor device |
| 109 | first secondary reference terminal of monitor device |
| 110 | first primary terminal of monitor device |
| 112 | first secondary terminal of monitor device |
| 114 | first tertiary terminal of monitor device |
| 116 | second primary terminal of monitor device |
| 118 | third primary terminal of monitor device |
| 120 | coupling part |
| 121 | power unit |
| 122 | antenna |
| 124 | wireless transceiver |
| 126 | loudspeaker |
| 128 | haptic feedback element |
| 140 | sensor unit |
| 150, 151 | proximal adhesive layer |
| 150A | distal surface of proximal adhesive layer |
| 150B | proximal surface of proximal adhesive layer |
| 200, | 201 first adhesive layer |
| 200A | distal surface of first adhesive layer |
| 200B | proximal surface of first adhesive layer |
| 202 | second adhesive layer |
| 202A | distal surface of second adhesive layer |
| 202B | proximal surface of second adhesive layer |
| 204, 500 | electrode assembly |
| 204A | distal surface of electrode assembly |
| 204B | proximal surface of electrode assembly |
| 206 | base release liner |
| 206A | distal surface of the release liner |
| 206B | proximal surface of the release liner |
| 208 | top layer |
| 208A | distal surface of the top layer |
| 208B | proximal surface of the top layer |
| 209 | coupling ring |
| 210 | coupling part of first connector |
| 211 | first connector |
| 212 | terminals of first connector |
| 213 | first intermediate element |
| 213A | distal surface of first intermediate element |
| 213B | proximal surface of first intermediate element |
| 214 | support layer of electrode assembly |
| 214A | distal surface of support layer |
| 214B | proximal surface of support layer |
| 216 | electrodes of electrode assembly |
| 217 | connection parts of electrodes |
| 218, 218A, 219 | masking layer / masking element |
| 218A | distal surface of masking element |
| 218B | proximal surface of masking element |
| 220, 220A, 220B | electrode configuration |
| 222 | first primary reference electrode |
| 222A | first primary reference connection part |
| 222B | sensing part of first primary reference electrode (first electrode branch) |

| | |
|---|---|
| 222C | ground conductor part |
| 222D | primary sensing part |
| 222E | tertiary sensing part |
| 222F | secondary sensing part |
| 224 | second primary electrode |
| 224A | second primary connection part |
| 224B | primary sensing part |
| 224C | second primary conductor part |
| 226 | third primary electrode |
| 226A | third primary connection part |
| 226B | primary sensing part |
| 226C | third primary conductor part |
| 228 | fourth primary electrode |
| 228A | fourth primary connection part |
| 228B | primary sensing part |
| 228C | fourth primary conductor part |
| 230 | first primary electrode |
| 230A | first primary connection part |
| 230B | sensing part of first primary electrode |
| 230D | primary sensing part |
| 230E | secondary sensing part |
| 232 | first secondary electrode |
| 232A | first secondary connection part |
| 232B | sensing part of first secondary electrode |
| 232D | secondary sensing part |
| 232E | tertiary sensing part |
| 234 | second electrode branch of the first primary reference electrode |
| 234B | primary sensing part |
| 236 | third electrode branch of the first primary reference electrode |
| 238 | fourth electrode branch of the first primary reference electrode |
| 240 | first electrode branch of the first primary reference electrode |
| 242 | ground terminal opening |
| 244 | first terminal opening |
| 246 | second terminal opening |
| 248 | third terminal opening |
| 250 | fourth terminal opening |
| 252 | fifth terminal opening |
| 254 | first primary sensor point openings of masking element |
| 254A | first primary sensor point opening for the first primary reference electrode |
| 254B | first primary sensor point opening for the first primary electrode |
| 256 | first secondary sensor point openings of masking element |
| 256A | first secondary sensor point opening for the first secondary electrode |
| 256B | first secondary sensor point opening for the first secondary electrode |
| 258 | first tertiary sensor point openings of masking element |
| 258A | first tertiary sensor point opening for the first secondary electrode |
| 258B | first tertiary sensor point opening for the first primary reference electrode |
| 260 | first primary sensor point openings of adhesive layer |
| 260A | first primary sensor point opening for the first primary reference electrode |
| 260B | first primary sensor point opening for the first primary electrode |
| 262 | first secondary sensor point openings of adhesive layer |
| 262A | first secondary sensor point opening for the first secondary electrode |
| 262B | first secondary sensor point opening for the first primary electrode |
| 264 | first tertiary sensor point openings of adhesive layer |
| 264A | first tertiary sensor point opening for the first secondary electrode |
| 264B | first tertiary sensor point opening for the first primary reference electrode |
| 282 | ground terminal element |
| 282A | ground terminal |
| 284 | first terminal element |

| 284A | first terminal |
|---|---|
| 286 | second terminal element |
| 286A | second terminal |
| 288 | third terminal element |
| 288A | third terminal |
| 290 | fourth terminal element |
| 290A | fourth terminal |
| 292 | fifth terminal element |
| 292A | fifth terminal |
| 400 | first primary sensing zone |
| 402 | frist secondary sensing zone |
| 404 | first tertiary sensing zone |
| 406 | first direction/zero direction |
| 408 | second direction |
| 410 | third direction |
| 412 | first tertiary electrode |
| 412B | tertiary sensing part |
| 414 | first quaternary electrode |
| 416 | first quaternary sensing zone |
| 418 | fourth direction |
| 504 | support member |
| 506 | base part |
| 510 | joint, weakened line |
| 512 | third primary sensor point opening |
| 520 | sensing zone configuration |
| 522 | second primary sensing zone |
| 524 | second secondary sensing zone |
| 526 | second tertiary sensing zone |
| D_1 | first distance |
| D_1_1 | first primary distance |
| D_1_2 | first secondary distance |
| D_1_3 | first tertiary |
| D_2 | second distance |
| D_2_1 | second primary distance |
| D_3_1 | third primary distance |
| D_4_1 | fourth primary distance |
| RG1 | first ground distance |
| RG2 | second ground distance |
| D_3 | third radial distance |
| RG3 | third ground distance |
| V_1_1 | first primary angle |
| V_1_2 | first secondary angle |
| V_1_3 | first tertiary angle |
| V_1_4 | first tertiary angle |
| V_2_1 | second primary angle |

**Claims**

1. An electrode assembly (204, 500) for a base plate of an ostomy appliance, the electrode assembly comprising:

- a support layer (214), the support layer having a stomal opening (18C) with a centre point (19); and
- a plurality of electrodes including a first set of electrodes, the first set of electrodes comprising a first primary electrode (230), a first secondary electrode (232), a first tertiary electrode (412), and one or more first reference electrodes including a first primary reference electrode (222),

wherein the plurality of electrodes is configured to detect presence of fluid in a first primary sensing zone (400), a first secondary sensing zone (402), and a first tertiary sensing zone (404), the first primary sensing zone (400) arranged in a first primary angle space (V_1_1) from the centre point of the support layer, the first secondary sensing zone (402) arranged in a first secondary angle space (V_1_2) from the centre point of the support layer, and the first tertiary sensing zone (404) arranged in a first tertiary angle space (V_1_3) from the centre point of the support layer; and

wherein the plurality of electrodes is further configured to detect presence of fluid in a second primary sensing zone (522), a second secondary sensing zone (524), and a second tertiary sensing zone (526), the second primary sensing zone (522) arranged in a second primary angle space from the centre point of the support layer, the second secondary sensing zone (524) arranged in a second secondary angle space from the centre point of the support layer, and the second tertiary sensing zone (526) arranged in a second tertiary angle space from the centre point of the support layer; and

**characterised in that** the first primary sensing zone (400) and the first secondary sensing zone (402) are separate sensing zones, and **in that** the second primary angle space partly overlaps the first tertiary angle space and/or the first secondary angle space, the second secondary angle space partly overlaps the first primary angle space and/or the first tertiary angle space, and the second tertiary angle space partly overlaps the first primary angle space and/or the first secondary angle space.

2. Electrode assembly according to claim 1, wherein the electrode assembly comprises a support member (504) comprising a base part (506) and a coupling part (210) tiltably attached to the base part via a joint.

3. Electrode assembly according to claim 2, wherein the coupling part is angled at least 15 degrees with respect to the base part.

4. Electrode assembly according to any of claims 1-3, wherein the first set of electrodes is configured to detect presence of fluid in a first quaternary sensing zone (416), the first quaternary sensing zone (416) arranged in a first quaternary angle space (V_1_4) from the centre point of the support layer.

5. Electrode assembly according to any of claims 1-4, wherein the first set of electrodes is configured to detect presence of fluid in a first quinary sensing zone, the first quinary sensing zone arranged in a first quinary angle space from the centre point of the support layer.

6. Electrode assembly according to any of claims 1-5, wherein the first set of electrodes is configured to detect presence of fluid in a first senary sensing zone, the first senary sensing zone arranged in a first senary angle space from the centre point of the support layer.

7. Electrode assembly according to any of claims 1-6, wherein the plurality of electrodes comprises a second set of electrodes, the second set of electrodes comprising a second primary electrode (224), and wherein the second primary electrode is configured to detect presence of fluid at a second primary distance from the centre point of the support layer.

8. Electrode assembly according to any of claims 1-7, wherein the electrode assembly comprises a masking layer (218) arranged on a proximal side of the support layer, the masking layer covering at least parts of electrodes of the electrode assembly for forming conductor parts of electrodes.

9. Electrode assembly according to any of claims 1-8, wherein the first primary angle space spans a first primary angle in the range from 45° to 315°, and/or the first secondary angle space spans a first secondary angle in the range from 45° to 315°, and/or the first tertiary angle space spans a first tertiary angle in the range from 45° to 180°.

10. Electrode assembly according to any of claims 1-9, wherein the first primary electrode (230) comprises a plurality of primary sensing parts (230D) arranged in the first primary sensing zone.

11. Electrode assembly according to any of claims 1-10, wherein the first secondary electrode (232) comprises a plurality of secondary sensing parts (232D) arranged in the first secondary sensing zone.

12. Electrode assembly according to any of claims 1-11, wherein the first tertiary electrode (412) comprises a plurality of tertiary sensing parts (412B) arranged in the first tertiary sensing zone.

13. A base plate (4) for an ostomy appliance (2), wherein the base plate (4) comprises an electrode assembly (204, 500) according to any of claims 1-12.

14. An ostomy appliance (2) comprising an electrode assembly (204, 500) according to any of claims 1-12.

**Patentansprüche**

1. Elektrodenanordnung (204, 500) für eine Basisplatte einer Stomavorrichtung, wobei die Elektrodenanordnung umfasst:

   - eine Trägerschicht (214), wobei die Trägerschicht eine Stomaöffnung (18C) mit einem Mittelpunkt (19) aufweist; und
   - mehrere Elektroden, was einen ersten Satz von Elektroden einschließt, wobei der erste Satz von Elektroden eine erste primäre Elektrode (230), eine erste sekundäre Elektrode (232), eine erste tertiäre Elektrode (412) und eine oder mehrere erste Referenzelektroden umfasst, was eine erste primäre Referenzelektrode (222) einschließt,
   wobei die mehreren Elektroden dazu ausgelegt sind, das Vorhandensein von Flüssigkeit in einer ersten primären Erfassungszone (400), einer ersten sekundären Erfassungszone (402) und einer ersten tertiären Erfassungszone (404) zu detektieren, wobei die erste primäre Erfassungszone (400) in einem ersten primären Winkelraum (V_1_1) vom Mittelpunkt der Trägerschicht aus angeordnet ist, die erste sekundäre Erfassungszone (402) in einem ersten sekundären Winkelraum (V_1_2) vom Mittelpunkt der Trägerschicht aus angeordnet ist und die erste tertiäre Erfassungszone (404) in einem ersten tertiären Winkelraum (V_1_3) vom Mittelpunkt der Trägerschicht aus angeordnet ist; und
   wobei die mehreren Elektroden ferner dazu ausgelegt sind, das Vorhandensein von Flüssigkeit in einer zweiten primären Erfassungszone (522), einer zweiten sekundären Erfassungszone (524) und einer zweiten tertiären Erfassungszone (526) zu detektieren, wobei die zweite primäre Erfassungszone (522) in einem zweiten primären Winkelraum vom Mittelpunkt der Trägerschicht aus angeordnet ist, die zweite sekundäre Erfassungszone (524) in einem zweiten sekundären Winkelraum vom Mittelpunkt der Trägerschicht aus angeordnet ist und die zweite tertiäre Erfassungszone (526) in einem zweiten tertiären Winkelraum vom Mittelpunkt der Trägerschicht aus angeordnet ist; und
   **dadurch gekennzeichnet, dass** die erste primäre Erfassungszone (400) und die erste sekundäre Erfassungszone (402) getrennte Erfassungszonen sind und dass der zweite primäre Winkelraum den ersten tertiären Winkelraum und/oder den ersten sekundären Winkelraum teilweise überlappt, der zweite sekundäre Winkelraum den ersten primären Winkelraum und/oder den ersten tertiären Winkelraum teilweise überlappt und der zweite tertiäre Winkelraum den ersten primären Winkelraum und/oder den ersten sekundären Winkelraum teilweise überlappt.

2. Elektrodenanordnung gemäß Anspruch 1, wobei die Elektrodenanordnung ein Trägerelement (504) umfasst, umfassend ein Basisteil (506) und ein Kopplungsteil (210), das über ein Gelenk kippbar an dem Basisteil angebracht ist.

3. Elektrodenanordnung gemäß Anspruch 2, wobei das Kopplungsteil in Bezug auf das Basisteil um wenigstens 15 Grad angewinkelt ist.

4. Elektrodenanordnung gemäß einem der Ansprüche 1-3, wobei der erste Satz von Elektroden dazu ausgelegt ist, das Vorhandensein von Flüssigkeit in einer ersten quaternären Erfassungszone (416) zu detektieren, wobei die erste quaternäre Erfassungszone (416) in einem ersten quaternären Winkelraum (V_1_4) vom Mittelpunkt der Trägerschicht aus angeordnet ist.

5. Elektrodenanordnung gemäß einem der Ansprüche 1-4, wobei der erste Satz von Elektroden dazu ausgelegt ist, das Vorhandensein von Flüssigkeit in einer ersten quinären Erfassungszone zu detektieren, wobei die erste quinäre Erfassungszone in einem ersten quinären Winkelraum vom Mittelpunkt der Trägerschicht aus angeordnet ist.

6. Elektrodenanordnung gemäß einem der Ansprüche 1-5, wobei der erste Satz von Elektroden dazu ausgelegt ist, das Vorhandensein von Flüssigkeit in einer ersten senären Erfassungszone zu detektieren, wobei die erste senäre Erfassungszone in einem ersten senären Winkelraum vom Mittelpunkt der Trägerschicht aus angeordnet ist.

7. Elektrodenanordnung gemäß einem der Ansprüche 1-6, wobei die mehreren Elektroden einen zweiten Satz von Elektroden umfassen, wobei der zweite Satz von Elektroden eine zweite primäre Elektrode (224) umfasst und wobei die zweite primäre Elektrode dazu ausgelegt ist, das Vorhandensein von Flüssigkeit in einem zweiten primären Abstand vom Mittelpunkt der Trägerschicht aus zu detektieren.

8. Elektrodenanordnung gemäß einem der Ansprüche 1-7, wobei die Elektrodenanordnung eine Maskierungsschicht (218) umfasst, die an einer proximalen Seite der Trägerschicht angeordnet ist, wobei die Maskierungsschicht wenigstens Teile von Elektroden der Elektrodenanordnung zum Bilden von Leiterteilen von Elektroden abdeckt.

9. Elektrodenanordnung gemäß einem der Ansprüche 1-8, wobei der erste primäre Winkelraum einen ersten primären Winkel im Bereich von 45° bis 315° umspannt und/oder der erste sekundäre Winkel einen ersten sekundären Winkel im Bereich von 45° bis 315° umspannt und/oder der erste tertiäre Winkel einen ersten tertiären Winkel im Bereich von 45° bis 180° umspannt.

10. Elektrodenanordnung gemäß einem der Ansprüche 1-9, wobei die erste primäre Elektrode (230) mehrere primäre Erfassungsteile (230D) umfasst, die in der ersten primären Erfassungszone angeordnet sind.

11. Elektrodenanordnung gemäß einem der Ansprüche 1-10, wobei die erste sekundäre Elektrode (232) mehrere sekundäre Erfassungsteile (232D) umfasst, die in der ersten sekundären Erfassungszone angeordnet sind.

12. Elektrodenanordnung gemäß einem der Ansprüche 1-11, wobei die erste tertiäre Elektrode (412) mehrere tertiäre Erfassungsteile (412B) umfasst, die in der ersten tertiären Erfassungszone angeordnet sind.

13. Basisplatte (4) für eine Stomavorrichtung (2), wobei die Basisplatte (4) eine Elektrodenanordnung (204, 500) gemäß einem der Ansprüche 1-12 umfasst.

14. Stomavorrichtung (2), umfassend eine Elektrodenanordnung (204, 500) gemäß einem der Ansprüche 1-12.

**Revendications**

1. Ensemble d'électrodes (204, 500) pour une plaque de base **d'un** appareil de stomie, l'ensemble **d'électrodes** comprenant :

   - une couche de support (214), la couche de support ayant une ouverture stomale (18C) avec un point central (19) ; et
   - une pluralité **d'électrodes** comportant un premier ensemble d'électrodes, le premier ensemble d'électrodes comprenant une première électrode primaire (230), une première électrode secondaire (232), une première électrode tertiaire (412), et une ou plusieurs premières électrodes de référence comportant une première électrode de référence primaire (222),
   dans lequel la pluralité d'électrodes est configurée pour détecter la présence de fluide dans une première zone de détection primaire (400), une première zone de détection secondaire (402) et une première zone de détection tertiaire (404), la première zone de détection primaire (400) étant agencée dans un premier espace angulaire primaire (V_1 1) à partir du point central de la couche de support, la première zone de détection secondaire (402) étant agencée dans un premier espace angulaire secondaire (V_1_2) à partir du point central de la couche de la couche de support, et la première zone de détection tertiaire (404) étant agencée dans un premier espace angulaire tertiaire (V_1 3) à partir du point central de la couche de support ; et
   dans lequel la pluralité d'électrodes est en outre configurée pour détecter la présence de fluide dans une seconde zone de détection primaire (522), une seconde zone de détection secondaire (524) et une seconde zone de détection tertiaire (526), la seconde zone de détection primaire (522) étant agencée dans un second espace angulaire primaire à partir du point central de la couche de support, la seconde zone de détection secondaire (524) étant agencée dans un second espace angulaire secondaire à partir du point central de la couche de support, et la seconde zone de détection tertiaire (526) étant agencée dans un second espace angulaire tertiaire à partir du point central de la couche de support ; et
   **caractérisé en ce que** la première zone de détection primaire (400) et la première zone de détection secondaire (402) sont des zones de détection distinctes, et **en ce que** le second espace angulaire primaire chevauche partiellement le premier espace angulaire tertiaire et/ou le premier espace angulaire secondaire, le second espace angulaire secondaire chevauche partiellement le premier espace angulaire primaire et/ou le premier espace angulaire tertiaire, et le second espace angulaire tertiaire chevauche partiellement le premier espace angulaire primaire et/ou le premier espace angulaire secondaire.

2. Ensemble d'électrodes selon la revendication **1,** dans lequel l'ensemble d'électrodes comprend un élément de support (504) comprenant une partie de base (506) et une partie **d'accouplement** (210) fixée de manière inclinable à la partie de base par l'intermédiaire d'un joint.

**3.** Ensemble d'électrodes selon la revendication **2,** dans lequel la partie **d'accouplement** est penchée **d'au** moins 15 degrés par rapport à la partie de base.

**4.** Ensemble **d'électrodes** selon l'une quelconque des revendications 1 à 3, dans lequel le premier ensemble d'électrodes est configuré pour détecter la présence de fluide dans une première zone de détection quaternaire (416), la première zone de détection quaternaire (416) étant agencée dans un premier espace angulaire quaternaire (V_1 4) à partir du point central de la couche de support.

**5.** Ensemble **d'électrodes** selon l'une quelconque des revendications 1 à 4, dans lequel le premier ensemble d'électrodes est configuré pour détecter la présence de fluide dans une première zone de détection quinaire, la première zone de détection quinaire étant agencée dans un premier espace angulaire quinaire à partir du point central de la couche de support.

**6.** Ensemble **d'électrodes** selon l'une quelconque des revendications 1 à 5, dans lequel le premier ensemble d'électrodes est configuré pour détecter la présence de fluide dans une première zone de détection sénaire, la première zone de détection sénaire étant agencée dans un premier espace angulaire sénaire à partir du point central de la couche de support.

**7.** Ensemble d'électrodes selon l'une quelconque des revendications 1 à 6, dans lequel la pluralité d'électrodes comprend un second ensemble d'électrodes, le second ensemble d'électrodes comprenant une seconde électrode primaire (224), et dans lequel la seconde électrode primaire est configurée pour détecter la présence de fluide à une seconde distance primaire du point central de la couche de support.

**8.** Ensemble **d'électrodes** selon l'une quelconque des revendications 1 à 7, dans lequel l'ensemble d'électrodes comprend une couche de masquage (218) agencée sur un côté proximal de la couche de support, la couche de masquage recouvrant au moins des parties d'électrodes de l'ensemble d'électrodes pour former des parties conductrices d'électrodes.

**9.** Ensemble d'électrodes selon l'une quelconque des revendications 1 à 8, dans lequel le premier espace angulaire primaire s'étend sur un premier angle primaire dans la plage de 45° à 315°, et/ou le premier espace angulaire secondaire s'étend sur un premier angle secondaire dans la plage de 45° à 315°, et/ou le premier espace angulaire tertiaire s'étend sur un premier angle tertiaire dans la plage de 45° à 180°.

**10.** Ensemble d'électrodes selon l'une quelconque des revendications 1 à 9, dans lequel la première électrode primaire (230) comprend une pluralité de parties de détection primaires (230D) agencées dans la première zone de détection primaire.

**11.** Ensemble d'électrodes selon l'une quelconque des revendications 1 à 10, dans lequel la première électrode secondaire (232) comprend une pluralité de parties de détection secondaires (232D) agencées dans la première zone de détection secondaire.

**12.** Ensemble d'électrodes selon l'une quelconque des revendications 1 à 11, dans lequel la première électrode tertiaire (412) comprend une pluralité de parties de détection tertiaires (412B) agencées dans la première zone de détection tertiaire.

**13.** Plaque de base (4) pour un appareil de stomie (2), dans laquelle la plaque de base (4) comprend un ensemble d'électrodes (204, 500) selon l'une quelconque des revendications 1 à 12.

**14.** Appareil de stomie (2) comprenant un ensemble d'électrodes (204, 500) selon l'une quelconque des revendications 1 à 12.

**Fig. 1**

Fig. 2

**Fig. 3**

204, 204B

218

218B

216

19

217

214

214B

18C

**Fig. 4**

**Fig. 5**

**Fig. 6**

Fig. 7

**Fig. 8**

**Fig. 9**

**Fig. 10**

Fig. 11

**Fig. 12**

**Fig. 13**

201, 151

200A, 150A

260

262

19

**Fig. 14**

**Fig. 15**

**Fig. 16**

**Fig. 17**

**Fig. 18**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019142623 A1